# EUROPEAN PATENT APPLICATION

(11) **EP 3 926 342 A1**
(43) Date of publication of application: **22.12.2021**
(21) Application number: 20180358.2
(22) Date of filing: 16.06.2020
(51) Int. Cl.: G01N 33/569, A61K 38/00, A61K 39/00, C07K 14/00, C07K 16/00

(54) **RHINOVIRUS ASSAY**

(71) Applicant: Viravaxx AG, 1090 Wien (AT)
(72) Inventor: VALENTA, Rudolf, 2604 Theresienfeld (AT); NIESPODZIANA, Katarzyna, 1210 Vienna (AT); PAZDEROVA, Petra, 90101 Malachky (SK); HENNING, RAINER, 8738 Uetliburg (CH); WALTL, Eva, 6372 Obendorf in Tirol (AT); NIEDERBERGER-LEPPIN, Verena, 1180 Vienna (AT); FLICKER, Sabine, 3400 Klosterneuburg (AT); FOCKE-TEJKL, Margarte, 1140 Vienna (AT)
(74) Representative: Redl, Gerda

(57) **Abstract**

The present invention relates to a method for determining the binding of a rhinovirus (RV) strain or fragment thereof to a rhinovirus receptor protein or functional fragment thereof comprising the steps of
a) incubating a solid support comprising at least one type of rhinovirus receptor protein or a functional fragment thereof immobilized thereon with a rhinovirus strain or fragment thereof, and
b) determining the binding level of said rhinovirus strain or fragment thereof to said rhinovirus receptor protein or functional fragment thereof,
wherein a detection molecule and/or a labelling molecule is used in step b) for determining said binding level.

SEQUENCE LISTING

<110>  Viravaxx AG

<120>  RHINOVIRUS ASSAY

<130>  27004-EP

<160>  14

<170>  PatentIn version 3.5

<210>  1
<211>  31
<212>  PRT
<213>  Artificial Sequence

<220>
<223>  Fragment of human ICAM-1

<400>  1

Gln Thr Ser Val Ser Pro Ser Lys Val Ile Leu Pro Arg Gly Gly Ser
1               5                   10                  15

Val Leu Val Thr Cys Ser Thr Ser Cys Asp Gln Pro Lys Leu Leu
            20                  25                  30

<210>  2
<211>  32
<212>  PRT
<213>  Artificial Sequence

<220>
<223>  Fragment of human ICAM-1

<400>  2

Ser Thr Ser Cys Asp Gln Pro Lys Leu Leu Gly Ile Glu Thr Pro Leu
1               5                   10                  15

Pro Lys Lys Glu Leu Leu Leu Pro Gly Asn Asn Arg Lys Val Tyr Glu
            20                  25                  30

<210>  3
<211>  34
<212>  PRT
<213>  Artificial Sequence

<220>
<223>  Fragment of human ICAM-1

<400>  3

Lys Lys Glu Leu Leu Leu Pro Gly Asn Asn Arg Lys Val Tyr Glu Leu
1               5                   10                  15

Ser Asn Val Gln Glu Asp Ser Gln Pro Met Cys Tyr Ser Asn Cys Pro

20                  25                  30

Asp Gly

<210>  4
<211>  32
<212>  PRT
<213>  Artificial Sequence

<220>
<223>  Fragment of human ICAM-1

<400>  4

Tyr Glu Leu Ser Asn Val Gln Glu Asp Ser Gln Pro Met Cys Tyr Ser
1               5                   10                  15

Asn Cys Pro Asp Gly Gln Ser Thr Ala Lys Thr Phe Leu Thr Val Tyr
            20                  25                  30

<210>  5
<211>  31
<212>  PRT
<213>  Artificial Sequence

<220>
<223>  Fragment of human ICAM-1

<400>  5

Trp Thr Pro Glu Arg Val Glu Leu Ala Pro Leu Pro Ser Trp Gln Pro
1               5                   10                  15

Val Gly Lys Asn Leu Thr Leu Arg Cys Gln Val Glu Gly Gly Ala
            20                  25                  30

<210>  6
<211>  35
<212>  PRT
<213>  Artificial Sequence

<220>
<223>  Fragment of human ICAM-1

<400>  6

Leu Val Arg Arg Asp His His Gly Ala Asn Phe Ser Cys Arg Thr Glu
1               5                   10                  15

Leu Asp Leu Arg Pro Gln Gly Leu Glu Leu Phe Glu Asn Thr Ser Ala
            20                  25                  30

Pro Tyr Gln
        35

<210>  7
<211>  38
<212>  PRT
<213>  Artificial Sequence

<220>
<223>  Fragment of human ICAM-1

<400>  7

Ala Leu Gly Asp Gln Arg Leu Asn Pro Thr Val Thr Tyr Gly Asn Asp
1               5                   10                  15

Ser Phe Ser Ala Lys Ala Ser Val Ser Val Thr Ala Glu Asp Glu Gly
            20                  25                  30

Thr Gln Arg Leu Thr Cys
        35

<210>  8
<211>  31
<212>  PRT
<213>  Artificial Sequence

<220>
<223>  Fragment of human ICAM-1

<400>  8

Glu Val Ser Glu Gly Thr Glu Val Thr Val Lys Cys Glu Ala His Pro
1               5                   10                  15

Arg Ala Lys Val Thr Leu Asn Gly Val Pro Ala Gln Pro Leu Gly
            20                  25                  30

<210>  9
<211>  34
<212>  PRT
<213>  Artificial Sequence

<220>
<223>  Fragment of human ICAM-1

<400>  9

Ala Thr Pro Glu Asp Asn Gly Arg Ser Phe Ser Cys Ser Ala Thr Leu
1               5                   10                  15

Glu Val Ala Gly Gln Leu Ile His Lys Asn Gln Thr Arg Glu Leu Arg
            20                  25                  30

Val Leu

<210>  10
<211>  37
<212>  PRT
<213>  Artificial Sequence

<220>
<223>  Fragment of human ICAM-1

<400>  10

Gly Pro Arg Leu Asp Glu Arg Asp Cys Pro Gly Asn Trp Thr Trp Pro
1               5                   10                  15

Glu Asn Ser Gln Gln Thr Pro Met Cys Gln Ala Trp Gly Asn Pro Leu
            20                  25                  30

Pro Glu Leu Lys Cys
        35

<210>  11
<211>  32
<212>  PRT
<213>  Artificial Sequence

<220>
<223>  Fragment of human ICAM-1

<400>  11

Val Thr Val Thr Arg Asp Leu Glu Gly Thr Tyr Leu Cys Arg Ala Arg
1               5                   10                  15

Ser Thr Gln Gly Glu Val Thr Arg Lys Val Thr Val Asn Val Leu Ser
            20                  25                  30

<210>  12
<211>  532
<212>  PRT
<213>  Homo sapiens

<400>  12

Met Ala Pro Ser Ser Pro Arg Pro Ala Leu Pro Ala Leu Leu Val Leu
1               5                   10                  15

Leu Gly Ala Leu Phe Pro Gly Pro Gly Asn Ala Gln Thr Ser Val Ser
            20                  25                  30

Pro Ser Lys Val Ile Leu Pro Arg Gly Gly Ser Val Leu Val Thr Cys
        35                  40                  45

Ser Thr Ser Cys Asp Gln Pro Lys Leu Leu Gly Ile Glu Thr Pro Leu
    50                  55                  60

Pro Lys Lys Glu Leu Leu Leu Pro Gly Asn Asn Arg Lys Val Tyr Glu
65                  70                  75                  80

Leu Ser Asn Val Gln Glu Asp Ser Gln Pro Met Cys Tyr Ser Asn Cys
                85                  90                  95

Pro Asp Gly Gln Ser Thr Ala Lys Thr Phe Leu Thr Val Tyr Trp Thr
            100                 105                 110

Pro Glu Arg Val Glu Leu Ala Pro Leu Pro Ser Trp Gln Pro Val Gly
        115                 120                 125

Lys Asn Leu Thr Leu Arg Cys Gln Val Glu Gly Gly Ala Pro Arg Ala
    130                 135                 140

Asn Leu Thr Val Val Leu Leu Arg Gly Glu Lys Glu Leu Lys Arg Glu
145                 150                 155                 160

Pro Ala Val Gly Glu Pro Ala Glu Val Thr Thr Thr Val Leu Val Arg
                165                 170                 175

Arg Asp His His Gly Ala Asn Phe Ser Cys Arg Thr Glu Leu Asp Leu
            180                 185                 190

Arg Pro Gln Gly Leu Glu Leu Phe Glu Asn Thr Ser Ala Pro Tyr Gln
        195                 200                 205

Leu Gln Thr Phe Val Leu Pro Ala Thr Pro Pro Gln Leu Val Ser Pro
    210                 215                 220

Arg Val Leu Glu Val Asp Thr Gln Gly Thr Val Val Cys Ser Leu Asp
225                 230                 235                 240

Gly Leu Phe Pro Val Ser Glu Ala Gln Val His Leu Ala Leu Gly Asp
                245                 250                 255

Gln Arg Leu Asn Pro Thr Val Thr Tyr Gly Asn Asp Ser Phe Ser Ala
            260                 265                 270

Lys Ala Ser Val Ser Val Thr Ala Glu Asp Glu Gly Thr Gln Arg Leu
        275                 280                 285

Thr Cys Ala Val Ile Leu Gly Asn Gln Ser Gln Glu Thr Leu Gln Thr
    290                 295                 300

Val Thr Ile Tyr Ser Phe Pro Ala Pro Asn Val Ile Leu Thr Lys Pro
305                 310                 315                 320

Glu Val Ser Glu Gly Thr Glu Val Thr Val Lys Cys Glu Ala His Pro
                325                 330                 335

Arg Ala Lys Val Thr Leu Asn Gly Val Pro Ala Gln Pro Leu Gly Pro
            340                 345                 350

Arg Ala Gln Leu Leu Leu Lys Ala Thr Pro Glu Asp Asn Gly Arg Ser
        355                 360                 365

Phe Ser Cys Ser Ala Thr Leu Glu Val Ala Gly Gln Leu Ile His Lys
    370                 375                 380

Asn Gln Thr Arg Glu Leu Arg Val Leu Tyr Gly Pro Arg Leu Asp Glu
385                 390                 395                 400

Arg Asp Cys Pro Gly Asn Trp Thr Trp Pro Glu Asn Ser Gln Gln Thr
                405                 410                 415

Pro Met Cys Gln Ala Trp Gly Asn Pro Leu Pro Glu Leu Lys Cys Leu
            420                 425                 430

Lys Asp Gly Thr Phe Pro Leu Pro Ile Gly Glu Ser Val Thr Val Thr
        435                 440                 445

Arg Asp Leu Glu Gly Thr Tyr Leu Cys Arg Ala Arg Ser Thr Gln Gly
    450                 455                 460

Glu Val Thr Arg Lys Val Thr Val Asn Val Leu Ser Pro Arg Tyr Glu
465                 470                 475                 480

Ile Val Ile Ile Thr Val Val Ala Ala Ala Val Ile Met Gly Thr Ala
                485                 490                 495

Gly Leu Ser Thr Tyr Leu Tyr Asn Arg Gln Arg Lys Ile Lys Lys Tyr
            500                 505                 510

Arg Leu Gln Gln Ala Gln Lys Gly Thr Pro Met Lys Pro Asn Thr Gln
        515                 520                 525

Ala Thr Pro Pro
    530

<210>  13
<211>  24
<212>  DNA
<213>  Artificial Sequence

<220>
<223>  Primer

<400>  13
gggacctact ttgggtgtcc gtgt                                              24


<210>  14
<211>  26
<212>  DNA
<213>  Artificial Sequence

<220>
<223>  Primer


<220>
<221>  misc_feature
<222>  (6)..(6)
<223>  n = inosine

<220>
<221>  misc_feature
<222>  (9)..(9)
<223>  n = C or T

<220>
<221>  misc_feature
<222>  (11)..(11)
<223>  n = A or G

<220>
<221>  misc_feature
<222>  (12)..(12)
<223>  n = A, G, C or T

<220>
<221>  misc_feature
<222>  (24)..(24)
<223>  n = A, G, C or T

<400>  14
gcatcnggna nnttccacca ccancc                                            26

## Description

### TECHNICAL FIELD

The present invention relates to a method for determining the binding of a rhinovirus strain to its receptor protein and to a method for identifying a compound for the treatment or prevention of a rhinovirus infection.

### BACKGROUND ART

Rhinovirus (RV) infections are the major cause for recurrent common colds and are responsible for more than 80% of wheeze and asthma exacerbations in children. Furthermore, RV-induced wheeze in early childhood seems to predispose children to develop recurrent wheeze and asthma later in life. Besides allergic sensitization, RV-infections are therefore among the most common trigger factors for asthma.

More than 160 different RV types are known. They can be divided in three RV species, RV-A, RV-B and RV-C according to their primary sequence and they are known to bind to defined receptors on their host cells. The majority of major group RVs belonging to RV-A and RV-B species bind to intercellular adhesion molecule 1 (ICAM-1) and a subset of RV-A species belonging to minor group RVs target low density lipoprotein receptor (LDLR). RV-C species, which according to sequence homology are more closely related to RV-A than to RV-B species, have been reported to use cadherin-related family member 3 (CDHR3) protein as receptor.

Several lines of evidence confirm the importance of RV infections as triggers for exacerbations of severe respiratory diseases such as childhood wheeze, asthma and chronic obstructive pulmonary disease. First of all, the association of RV infection and respiratory disease exacerbations has been demonstrated by the presence of virus in the respiratory tract of patients experiencing the exacerbations by showing the presence of virus-derived nucleic acids. Furthermore, it has been shown in experimental infection models that controlled infection with RV can indeed trigger asthma attacks. In particular, subjects suffering from allergic sensitizations seem to be especially susceptible to RV-triggered asthma, which may be related to the fact that their Th-2-biased immune system can less well combat RV infections.

The most common manifestation of rhinovirus, the common cold, is mild and self-limited. However, severe respiratory disease, including bronchiolitis and pneumonia, may occur as well.

The majority of RV-specific antibodies which develop in the course of RV infections are directed against a N-terminal peptide of the VP1 capsid protein (Niespodziana et al., The FASEB J. 26 (3): 1001-1008 (2012)). This finding not only allows to dissect RV species-specific antibody responses but also to relate the increases of RV-species-specific antibody responses to respiratory disease exacerbations.

While substantial progress regarding the species-specific detection and diagnosis of RV infections by nucleic acid-based testing and antibody testing has been made, there is still no vaccine or RV-specific treatment available, which could be used for the prevention or treatment of RV-triggered respiratory illness. The development of RV-specific treatments and vaccines is challenging due to the occurrence of a large variety of RV-types.

Moreover, a systematic development of vaccines, therapeutic antibodies and compounds which inhibit the infection of the host cell by the virus requires the availability of preclinical models, which allow the fast and high-throughput screening of candidate molecules. Regarding RV, there are currently only quite cumbersome and crude cell-based neutralization assays (Conant and Hamparin, J. Immunol. 100(1): 107-113 (1968), Edlmayr et al., Eur. Respir. J. 37 (1): 44-52 (2011)) available as well as mouse models which are based on transgenic animals expressing the human receptors (e.g., ICAM-1 knock-in mice) (Bartlett et al. in Rhinoviruses: pp 181-188, Springer (2015)).

It is an object of the present invention to provide a robust, simple and sensitive model for studying the interaction of rhinovirus species and their receptor proteins, which further allows to identify compounds inhibiting said interaction which can be used in the treatment or prevention of rhinovirus infections.

### SUMMARY OF THE INVENTION

Thus, the present invention relates to a method for determining the binding of a rhinovirus (RV) strain or fragment thereof to a rhinovirus receptor protein or functional fragment thereof comprising the steps of
a) incubating a solid support comprising at least one type of rhinovirus receptor protein or a functional fragment thereof immobilized thereon with a rhinovirus strain or fragment thereof, and
b) determining the binding level of said rhinovirus strain or fragment thereof to said rhinovirus receptor protein or functional fragment thereof,
   wherein a detection molecule and/or a labelling molecule is used in step b) for determining said binding level.

It surprisingly turned out that the method according to the present invention is more sensitive than conventional cell-based virus neutralization assays. In fact, a 20-fold increase in sensitivity was found when using the inventive method as compared to a conventional cell-based virus neutralization assay. The inventive method moreover allows to directly determine the binding of a RV to its receptor protein without the need of cells and is therefore faster and simpler than a conventional cell-based virus neutralization assay. The method of the present invention allows also to directly detect and identify virus neutralizing antibodies.

The method according to the present invention is particularly suitable for studying compounds influencing the RV-receptor binding. In particular, the present invention allows identifying a compound inhibiting the binding of a RV strain or fragment thereof to a RV receptor protein or functional fragment thereof. With the inventive method, a large number of compounds, which possibly inhibit RV-receptor binding can be tested.

Thus, the present invention also relates to a method for identifying a compound for the treatment or prevention of a rhinovirus infection comprising the steps of
a) incubating a first solid support comprising at least one type of a rhinovirus receptor protein or a functional fragment thereof immobilized thereon with a rhinovirus strain or fragment thereof and a first amount of said compound;
b) incubating a second solid support comprising at least one type of a rhinovirus receptor protein or a functional fragment thereof immobilized thereon with a rhinovirus strain or fragment thereof and a second amount of said compound, wherein said second amount of said compound is lower than the first amount of step a),
c) determining the levels of said rhinovirus strain or fragment thereof binding to said rhinovirus receptor protein or functional fragment thereof in the presence of the first and the second amount of said compound;
d) comparing the binding levels determined in step c), and
e) identifying said compound as rhinovirus infection treating or preventing compound if the binding level in the presence of the first amount of said compound is lower compared to the binding level in the presence of the second amount of said compound.

The present invention further relates to a kit for identifying a compound for the treatment or prevention of rhinovirus infections comprising
a) a solid support coated with at least one type of rhinovirus receptor protein or a functional fragment thereof;
b) at least one rhinovirus strain or a fragment thereof; and
c) at least one detection and/or labelling molecule.

The use of such kit enables straightforward identification of a compound for the treatment or prevention of rhinovirus infections.

Another aspect of the present invention relates to a composition for the use in the treatment or prevention of viral diseases comprising at least one fragment derived from amino acid residues 28 to 110 and/or from amino acid residues 390 to 480 of ICAM-1, preferably of human ICAM-1 (UniProt accession number P05362), at least one variant of said at least one fragment or at least one antibody or functional fragment thereof binding to said at least one fragment, wherein said at least one fragment consists of 20 to 80 amino acid residues.

It turned surprisingly out that fragments of the N- and C-terminal end of the ICAM-1 molecule induce the formation of antibodies able to block the binding of viruses or other pathogenic molecules and particles to ICAM-1. In particular, the finding that the C-terminal end of the ICAM-1 protein is involved in the binding of ICAM-1 to viruses or other pathogenic molecules is unexpected.

The present invention further relates to an antibody specific for a C-terminal peptide of ICAM-1 for use in the treatment or prevention of a rhinovirus infection.

### DESCRIPTION OF EMBODIMENTS

The present invention provides a method for determining the binding of a rhinovirus strain or fragment thereof to a rhinovirus receptor protein or functional fragment thereof comprising the steps of
a) incubating a solid support comprising at least one type of rhinovirus receptor protein or a functional fragment thereof immobilized thereon with a rhinovirus strain or fragment thereof, and
b) determining the binding level of said rhinovirus strain or fragment thereof to said rhinovirus receptor protein or functional fragment thereof,
   wherein a detection molecule and/or a labelling molecule is used in step b) for determining said binding level.

In a preferred embodiment, a compound inhibiting the binding of said rhinovirus strain or fragment thereof to said rhinovirus receptor protein or functional fragment thereof is present in step a). Such compound is useful for the treatment or prevention of rhinovirus infections.

Said compound may be a molecule binding to a rhinovirus strain or fragment thereof or it may be a molecule binding to a rhinovirus receptor protein or functional fragment thereof.

If said compound is a molecule binding to said rhinovirus strain or fragment thereof, the rhinovirus strain or fragment thereof is preferably pre-incubated with said compound prior to step a).

If said compound is a molecule binding to said receptor protein or functional fragment thereof, said receptor protein or functional fragment thereof is preferably pre-incubated with said compound prior to step a).

The present invention further provides a method for identifying a compound for the treatment or prevention of a rhinovirus infection comprising the steps of
a) incubating a first solid support comprising at least one type of a rhinovirus receptor protein or a functional fragment thereof immobilized thereon with a rhinovirus strain or fragment thereof and a first amount of said compound;
b) incubating a second solid support comprising at least one type of a rhinovirus receptor protein or a functional fragment thereof immobilized thereon with a rhinovirus strain or fragment thereof and a second amount of said compound, wherein said second amount of said compound is lower than the first amount of step a),
c) determining the levels of said rhinovirus strain or fragment thereof binding to said rhinovirus receptor protein or functional fragment thereof in the presence of the first and the second amount of said compound;
d) comparing the binding levels determined in step c), and
e) identifying said compound as rhinovirus infection treating or preventing compound if the binding level in the presence of the first amount of said compound is lower compared to the binding level in the presence of the second amount of said compound.

As used herein, the phrase "a compound for the treatment or prevention of a rhinovirus infection" refers to a compound, which inhibits the binding of a rhinovirus strain or fragment thereof to its receptor protein or a functional fragment thereof. The inventive methods allow observing such inhibition by determining the binding level of a rhinovirus strain or fragment thereof to its receptor protein or functional fragment thereof. Due to the inhibition of the virus-receptor binding the attachment of a virus particle on the surface of a cell can be prevented or at least significantly reduced. In consequence, no or a significantly reduced amount of virus particles is able to pass the cell barrier.

The phrase "determining the binding" or "determining the binding level" or "determining the level of binding" as used herein refers to quantifying the amount of rhinoviruses or fragments thereof that bind to the receptor protein or functional fragment thereof. The phrase "binding of a rhinovirus strain or fragment thereof to its receptor protein or functional fragment thereof" is also referred to as "RV-receptor binding" throughout the present specification.

A compound inhibits RV-receptor binding when a reduced binding level is determined in the presence of said compound when compared to the binding level determined in the presence of a lower amount of said compound, or in the absence of said compound.

Thus, according to the inventive methods, a compound inhibiting RV-receptor binding can be identified surprisingly fast and effectively with high sensitivity. Therefore, the present invention allows identifying compounds useful for the treatment or prevention of RV infections.

The term "rhinovirus strain or fragment thereof" as used herein refers to a strain from rhinovirus species A (RV-A), rhinovirus species B (RV-B) or rhinovirus species C (RV-C). Specifically, the RV strain may be a major group or minor group RV-A or RV-B strain. The RV strain may be a major group RV-A strain such as RV89, RV8, RV16, RV18, RV54. The RV strain may also be a minor group RV-A strain such as RV2, RV25, RV29 and RV44. The strain may further be a major group RV-B strain, such as RV14 or RV72. According to the present invention, the rhinovirus strain is therefore preferably selected from the group consisting of RV89, RV8, RV16, RV18, RV54, RV2, RV25, RV29, RV44, RV14 and RV72. Even more preferably, the rhinovirus strain is selected from the group consisting of RV89, RV2 and RV14.

The term "fragment of a rhinovirus strain" as used herein refers to a fragment of less than the full length of the proteins making up the capsid of the rhinovirus strain. Preferably, said fragment is a capsid protein, which preferably comprises the binding domain of the rhinovirus strain to its receptor ("rhinovirus-receptor binding domain"). Preferably, said fragment is selected from the group consisting of the capsid proteins VP1, VP2, VP3 and VP4, and any subfragment thereof comprising the binding domain of a RV strain to its receptor. Throughout this specification, the term "rhinovirus strain" includes any fragments thereof, if such fragments are not mentioned explicitly.

A "subfragment of a capsid protein", as used herein, is preferably a polypeptide comprising 10 to 150, preferably 10 to 100, consecutive amino acid residues of capsid protein VP1, VP2, VP3 or VP4. The "subfragment of a capsid protein" comprises preferably a rhinovirus-receptor binding domain.

According to the present invention, a "rhinovirus receptor protein" preferably refers to a human rhinovirus receptor protein. A "rhinovirus receptor protein" can be any protein or polypeptide naturally present on the surface of a cell, which is able to bind to a rhinovirus (particle) or a fragment thereof. Specifically, the rhinovirus receptor protein can be selected from the group consisting of intercellular adhesion molecule 1 (ICAM-1), low density lipoprotein receptor (LDLR), cadherin-related family member 3 (CDHR3), or a functional fragment thereof. Major group RV-A and RV-B strains are known to use intercellular adhesion molecule 1 (ICAM-1) as receptor protein. Minor group RV-A and RV-B strains are known to use low density lipoprotein receptor (LDLR). RV-C strains are known to use cadherin-related family member 3 (CDHR3) as receptor protein.

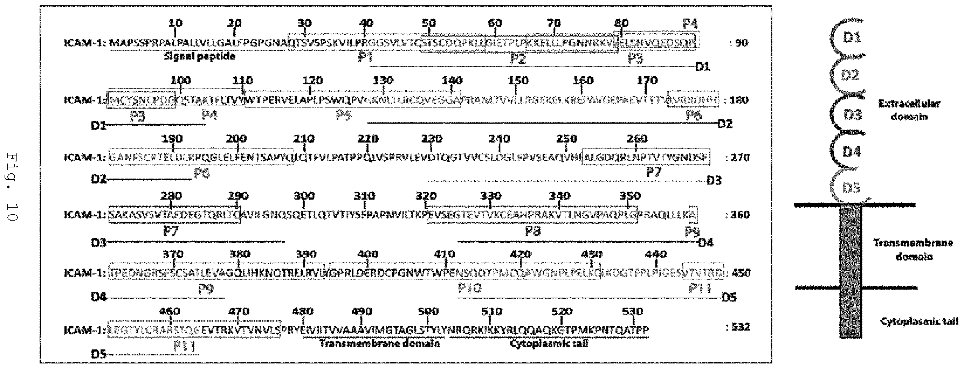

ICAM-1 used in the present invention can be a mammalian or human ICAM-1. In a preferred embodiment of the present invention, ICAM-1 is human ICAM-1 (UniProt accession number P05362). The human ICAM-1 protein comprises 532 amino acids, with a signal protein from amino acid residue 1 to 27, a transmembrane domain from amino acid residue 480 to 503 and a cytoplasmic tail from amino acid residue 504 to 532 (see Fig. 10). In one embodiment, said peptide derived from ICAM-1 may also be derived from other sources such as a mammalian source. Particularly preferred is ICAM-1 from horses, dogs, cats or camels.

The term "functional fragment of a rhinovirus receptor protein" as used herein refers to a fragment of less than the full length of the rhinovirus receptor protein, which maintains at least the rhinovirus specific binding function of the rhinovirus receptor protein and, thus, comprises at least the binding domain of the rhinovirus receptor protein to a rhinovirus strain or fragment thereof. Throughout this specification, the term "rhinovirus receptor protein" includes any functional fragments thereof, if such fragments are not mentioned explicitly.

According to the present invention, a solid support comprising at least one type of rhinovirus receptor protein or a functional fragment thereof immobilized thereon is incubated with a rhinovirus strain or fragment thereof. The solid support may be any solid carrier suitable for immobilizing a receptor protein. The solid support may be of any suitable shape, e.g. flat or spherical, or material, e.g. polymer or glass. Preferably, the solid support is a plate for performing an enzyme-linked immunosorbent assay (ELISA). The solid support may also be sepharose beads or nitrocellulose. On said solid support, at least one type of rhinovirus receptor protein or a functional fragment is immobilized. In one aspect, ICAM-1 or a functional fragment thereof is immobilized on said solid support. In another aspect, LDLR or a functional fragment thereof is immobilized on said solid support. In yet another aspect, CDHR3 or a functional fragment thereof is immobilized on said solid support. In yet another aspect, more than one type of receptor protein is immobilized on said solid support, for example ICAM-1 and LDLR, or functional fragments thereof.

According to the present invention, the RV-receptor binding level is determined. For determining said binding level, a detection molecule or labelling molecule may be used according to the present invention. In a preferred embodiment of the present invention, an enzyme-linked immunosorbent assay (ELISA) interaction assay is performed.

As used herein, the term "detection molecule" refers to a molecule, which binds to receptor-bound virus and allows quantifying the amount of virus or fragment thereof bound to its receptor protein or functional fragment thereof. Said detection molecule may be called a "first detection molecule" and may be combined with one or more further detection molecules. An anti-rhinovirus-antibody can be used as a first detection molecule, preferably in combination with a secondary detection molecule. Said secondary detection molecule may be an antibody binding to said first detection molecule and may be labelled with an enzyme, wherein said enzyme converts a substrate and thereby induces a colour reaction. Such colour reaction may be analysed spectroscopically to quantify the amount of rhinovirus strain or fragment thereof binding to its receptor.

In a specific embodiment of the present invention, the first detection molecule is a RV-specific antibody, preferably a RV-specific guinea pig (GP) antibody, and the secondary detection molecule is an antibody directed to said first detection molecule, preferably an anti-GP antibody, labelled with an enzyme such as horseradish peroxidase (HRP).

In a preferred embodiment, the detection molecule is an anti-rhinovirus antibody or fragment thereof, which binds to said rhinovirus strain or fragment thereof outside the rhinovirus-receptor binding domain. In other words, the detection molecule is an anti-rhinovirus antibody or fragment thereof, which does not inhibit the binding of said rhinovirus strain or fragment thereof to said rhinovirus receptor protein or functional fragment thereof. In a particularly preferred embodiment, said anti-rhinovirus antibody binding to rhinovirus outside the RV-receptor binding domain is an antibody binding to the N-terminus of a rhinovirus capsid protein such as the rhinovirus VP1 protein. The technical effect of using as a detection molecule an anti-rhinovirus antibody binding to rhinovirus outside the RV-receptor binding domain is an increased detection sensitivity, since any interference of the detection molecule with RV-receptor binding is avoided. In other words, the detection molecule does not itself inhibit the RV-receptor binding, which is advantageous since such inhibition would distort the measurement accuracy of the binding level.

Alternatively, the RV-receptor binding may be quantified using a labelling molecule. As used herein, a "labelling molecule" refers to a molecule, which is covalently linked to said rhinovirus strain or fragment thereof. In a specific embodiment, the labelling molecule is biotin and the binding level is determined by quantifying the amount of biotin-labelled receptor-bound rhinovirus. Said quantification may for example be performed by using Streptavidin-horseradish peroxidase (HRP) or Avidin-HRP protein conjugates, which specifically react with biotin and induce a colour reaction. The technical effect of using a labelling molecule for the detection is that RV-receptor binding can be monitored directly without the need of a secondary detection molecule. According to the present invention, the covalent bond between the labelling molecule and the rhinovirus strain is located outside the RV-receptor binding domain to avoid any interference of the labelling molecule with RV-receptor binding. Thus, high measurement sensitivity and accuracy of the binding level is achieved.

In a preferred embodiment, the rhinovirus strain or fragment thereof is pre-incubated with said compound prior to step a) of the inventive method for identifying a compound for the treatment or prevention of a rhinovirus infection.

In another preferred embodiment, the rhinovirus receptor protein or functional fragment thereof immobilized on said solid support is pre-incubated with said compound prior to step a) of the inventive method for identifying a compound for the treatment or prevention of a rhinovirus infection.

The compound for the treatment or prevention of a rhinovirus infection according to the present invention acts by inhibiting RV-receptor binding. Therefore, said compound may either bind to a rhinovirus strain or to its receptor protein. Specifically, said compound may bind to the RV-receptor binding domain in a rhinovirus strain. Alternatively, said compound may bind to the RV-binding domain in a rhinovirus receptor protein.

If a compound inhibiting RV-receptor binding is bound to a rhinovirus strain or to a rhinovirus receptor protein, the RV-receptor binding is inhibited and a reduced binding level is observed.

In some embodiments of the present invention, said compound is therefore a molecule binding to said rhinovirus strain or fragment thereof. In these embodiments, the rhinovirus strain or fragment thereof is preferably pre-incubated with said compound prior to step a) of the inventive method for identifying a compound for the treatment or prevention of a rhinovirus infection. The compound of the present invention may be a peptidic molecule (peptide, polypeptide or protein) or any other organic molecule (e.g. small molecule). Preferably, said compound is an antibody or a fragment thereof binding to said rhinovirus strain or fragment thereof.

In a particularly preferred embodiment, said compound is a rhinovirus-neutralizing antibody. The rhinovirus-neutralizing antibody may be an anti-VPl or anti-VP2 antibody. Preferably, the rhinovirus-neutralizing antibody is selected from the group consisting of human anti-VPl antibodies, humanized anti-VPl antibodies and rabbit anti-VPl antibodies. Even more preferably, said rhinovirus-neutralizing antibody is a human or humanized anti-VPl antibody. In another preferred embodiment, said rhinovirus-neutralizing antibody is selected from the group consisting of human anti-VP2 antibodies, humanized anti-VP2 antibodies and rabbit anti-VP2 antibodies.

In a further embodiment, said compound is a rhinovirus receptor protein or functional fragment thereof, preferably a recombinant rhinovirus receptor protein or functional fragment thereof.

In other embodiments, said compound is a molecule binding to said rhinovirus receptor protein or functional fragment thereof. In these embodiments, the rhinovirus receptor protein or functional fragment thereof immobilized on the solid support is preferably pre-incubated with said compound prior to step a) of the inventive method for identifying a compound for the treatment or prevention of a rhinovirus infection.

In one embodiment, said compound is an antibody selected from the group consisting of anti-ICAM-1 antibodies, anti-LDLR antibodies and functional fragments thereof.

In a further embodiment, said compound is selected from the group consisting of human anti-ICAM-1 antibodies, humanized anti-ICAM 1 antibodies and rabbit anti-ICAM-1 antibodies. Even more preferably, said compound is a human or humanized anti-ICAM-1 antibody.

In a preferred embodiment, said compound is an anti-ICAM-1 antibody directed against an N-terminal ICAM-1 peptide. Herein, the term "N-terminal ICAM-1 peptide" refers to a peptide derived from amino acid residues 28 to 110 of human ICAM-1 (UniProt accession number: P05362) or its corresponding region of a mammalian (e.g. horse, dog, cat, camel) ICAM-1 molecule. Said peptide may comprise or consist of 10 to 100, preferably 10 to 60, more preferably 10 to 40, amino acid residues. Preferably, said N-terminal ICAM-1 peptide consists of an amino acid sequence selected from the group consisting of SEQ ID No. 1 (QTSVSPSKVILPRGGSVLVTCSTSCDQPKLL), SEQ ID No. 2 (STSCDQPKLLGIETPLPKKELLLPGNNRKVYE), SEQ ID No. 3 (KKELLLPGNNRKVYELSNVQEDSQPMCYSNCPDG), SEQ ID No. 4 (YELSNVQEDSQPMCYSNCPDGQSTAKTFLTVY). Therefore, in one embodiment of the present invention, said compound is an antibody specific for an ICAM-1 peptide consisting of an amino acid sequence selected from the group consisting of SEQ ID No. 1, SEQ ID No. 2, SEQ ID No. 3 and SEQ ID No. 4.

In a further preferred embodiment, said compound is an anti-ICAM-1 antibody directed against a C-terminal ICAM-1 peptide. Herein, the term "C-terminal ICAM-1 peptide" refers to a peptide derived from amino acid residues 390 to 480 of human ICAM-1 (UniProt accession number: P05362) or its corresponding region of a mammalian (e.g. horse, dog, cat, camel) ICAM-1 molecule. In one embodiment, said peptide is derived from the D5 domain of the ICAM-1 protein. Preferably, said C-terminal ICAM-1 peptide consists of an amino acid sequence of SEQ ID No. 10 (GPRLDERDCPGNWTWPENSQQTPMCQAWGNPLPELKC). Therefore, in one embodiment of the present invention, said compound is an antibody specific for an ICAM-1 peptide with an amino acid sequence of SEQ ID No. 10. Surprisingly, the methods according to the present invention have allowed discovering that antibodies against a C-terminal ICAM-1 peptide inhibit the binding of rhinoviruses to ICAM-1. The present invention can therefore be used for identifying a compound for the treatment or prevention of a rhinovirus infection, which compound has not been described for such purpose before.

Therefore, the present invention also relates to an antibody specific for a C-terminal ICAM-1 peptide for use in the treatment or prevention of a rhinovirus infection. In a specific embodiment, said antibody specific for a C-terminal ICAM-1 peptide is used in a vaccine against a rhinovirus induced disease.

In a specific embodiment of the inventive methods a bioassay is performed, wherein said bioassay is preferably an enzyme-linked immunosorbent assay (ELISA), or an assay based on a solid support selected from the group consisting of sepharose beads and nitrocellulose.

The present invention further provides a kit for identifying a compound for the treatment or prevention of rhinovirus infections comprising
a) a solid support coated with at least one type of rhinovirus receptor protein or a functional fragment thereof;
b) at least one rhinovirus strain or a fragment thereof; and
c) at least one detection and/or labelling molecule.

In one embodiment of the inventive kit, the at least one type of rhinovirus receptor protein is intercellular adhesion molecule 1 (ICAM-1), low density lipoprotein receptor (LDLR), cadherin-related family member 3 (CDHR3), or a functional fragment thereof.

In a preferred embodiment, said at least one detection molecule is an anti-rhinovirus detection antibody, preferably combined with an enzyme-labelled secondary detection antibody, wherein said anti-rhinovirus detection antibody does not inhibit the binding of said rhinovirus strain or fragment thereof to said rhinovirus receptor protein or functional fragment thereof.

In another preferred embodiment, said at least one labelling molecule is covalently linked to said rhinovirus strain or fragment thereof. Preferably, said labelling molecule is biotin.

The present invention further refers to the use of an inventive kit for identifying compounds for the treatment and prevention of rhinovirus infections.

The present invention also provides a composition for the use in the treatment or prevention of viral diseases comprising at least one fragment derived from amino acid residues 28 to 110 and/or from amino acid residues 390 to 480 of ICAM-1, preferably of human ICAM-1 (see UniProt accession number: P05362), at least one variant of said at least one fragment or at least one antibody or functional fragment thereof binding to said at least one fragment, wherein said at least one fragment consists of 20 to 80 amino acid residues. Preferably, said composition for the use in the treatment or prevention of viral diseases comprises at least one fragment derived from amino acid residues 390 to 480 of ICAM-1, preferably of human ICAM-1 (UniProt accession number P05362), at least one variant of said at least one fragment or at least one antibody or functional fragment thereof binding to said at least one fragment, wherein said at least one fragment consists of 20 to 80 amino acid residues.

Herein, the term "variant" refers to a peptide with an amino acid sequence that differs by one or more amino acid residues from a fragment derived from amino acid residues 28 to 110 and/or from amino acid residues 390 to 480 of ICAM-1. The "variant" may have "conservative" amino acid changes, wherein a substituted amino acid has similar structural or chemical properties. One type of conservative amino acid substitutions refer to the interchangeability of residues having similar side chains. For example, a group of amino acids having aliphatic side chains is glycine, alanine, valine, leucine, and isoleucine; a group of amino acids having aliphatic-hydroxyl or sulfur-containing side chains is serine, threonine, cysteine and methionine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine. Preferred conservative amino acids substitution groups are: cysteine-serine, valine-leucine-isoleucine, phenylalanine-tyrosine, lysine-arginine, alanine-valine, and asparagine-glutamine. Most preferably, the conservative amino acid substitution is cysteine-serine. More rarely, a "variant" may have "nonconservative" changes (for example, replacement of a glycine with a tryptophan).

The "homologs" or "variants" of the present invention may have at least one, at least two, at least three, at least four, at least five, at least six, at least seven, at least eight, at least nine, or ten amino acid changes (substitution or deletion) compared to a peptide derived from amino acid residues 28 to 110 and/or from amino acid residues 390 to 480 of ICAM-1.

The composition for the use in the treatment or prevention of viral diseases according to the present invention may comprise at least one antibody or functional fragment thereof binding to said at least one fragment derived from amino acid residues 28 to 110 and/or from amino acid residues 390 to 480 of ICAM-1. The term "functional fragment" of an antibody as used herein refers to a fragment of less than the full length of the antibody, which maintains at least the binding function of the antibody to the fragment derived from amino acid residues 28 to 110 and/or from amino acid residues 390 to 480 of ICAM-1 and, thus, comprises at least the binding domain of the antibody to said fragment.

In one embodiment, the at least one fragment derived from amino acid residues 28 to 110 and/or from amino acid residues 390 to 480 of ICAM-1 or a variant thereof is fused or conjugated to a carrier protein.

"Fused", as used herein, refers to a protein comprising said fragment or variant thereof and said carrier protein that are expressed and prepared as one single recombinant polypeptide chain. Methods for the production of fusion proteins are well known in the art and can be found in standard molecular biology references such as Sambrook et al. (Molecular Cloning, 2nd ed., Cold Spring Harbor Laboratory Press, 1989) and Ausubel et al. (Short Protocols in Molecular Biology, 3rd ed; Wiley and Sons, 1995). In general, a fusion protein is produced by first constructing a fusion gene which is inserted into a suitable expression vector, which is, in turn, used to transfect a suitable host cell. In general, recombinant fusion constructs are produced by a series of restriction enzyme digestions and ligation reactions which result in the desired sequences being incorporated into a plasmid. If suitable restriction sites are not available, synthetic oligonucleotide adapters or linkers can be used as is known by those skilled in the art and described in the references cited above. The polynucleotide sequences encoding allergens and native proteins can be assembled prior to insertion into a suitable vector or the sequence encoding the allergen can be inserted adjacent to a sequence encoding a native sequence already present in a vector. Insertion of the sequence within the vector should be in frame so that the sequence can be transcribed into a protein. It will be apparent to those of ordinary skill in the art that the precise restriction enzymes, linkers and/or adaptors required as well as the precise reaction conditions will vary with the sequences and cloning vectors used. The assembly of DNA constructs, however, is routine in the art and can be readily accomplished by a person skilled in the art.

In a preferred embodiment, the least one fragment derived from amino acid residues 28 to 110 of ICAM-1 comprises or consists of an amino acid sequence selected from the group consisting of SEQ ID No. 1, SEQ ID No. 2, SEQ ID No. 3 and SEQ ID No. 4.

In another preferred embodiment, the at least one fragment derived from amino acid residues 390 to 480 of ICAM-1 comprises or consists of SEQ ID No. 10.

In a specific embodiment, the composition according to the present invention further comprises adjuvants and pharmaceutically acceptable excipients.

The composition according to the present invention is preferably used in the treatment or prevention of viral diseases in humans and ICAM-1 is preferably human ICAM-1. In another embodiment, the composition according to the present invention is used in the treatment or prevention of viral diseases in mammals such as equids, dogs and cats. In these embodiments, ICAM-1 is preferably the respective mammalian ICAM-1 and the inventive composition comprises at least one fragment derived from amino acid residues at the corresponding positions as amino acid residues 28 to 110 and/or amino acid residues 390 to 480 in human ICAM-1 (UniProt accession number: P05362) when the mammalian and human ICAM-1 are aligned using the Clustal method of alignment, wherein the default parameters for multiple alignment are GAP PENALTY=10, GAP LENGTH PENALTY=10 and default parameters for pairwise alignments are KTUPLE 1, GAP PENALTY=3, WINDOW=5 and DIAGONALS SAVED=5 (Higgins and Sharp (1989) CABIOS. 5:151-153). Said composition may also comprise at least one variant of said at least one fragment or at least one antibody or functional fragment thereof binding to said at least one fragment, wherein said at least one fragment consists of 20 to 80 amino acid residues.

The present invention also provides a compound comprising a peptide consisting of an amino acid sequence selected from the group consisting of SEQ ID No. 1, SEQ ID No. 2, SEQ ID No. 3, SEQ ID No. 4 and SEQ ID No. 10.

The present invention is further illustrated by the following embodiments, figures and examples, however, without being restricted thereto.

### Preferred embodiments:

1. A method for determining the binding of a rhinovirus strain or fragment thereof to a rhinovirus receptor protein or functional fragment thereof comprising the steps of
   a) incubating a solid support comprising at least one type of rhinovirus receptor protein or a functional fragment thereof immobilized thereon with a rhinovirus strain or fragment thereof, and
   b) determining the binding level of said rhinovirus strain or fragment thereof to said rhinovirus receptor protein or functional fragment thereof,
   wherein a detection molecule and/or a labelling molecule is used in step b) for determining said binding level.
2. The method of embodiment 1, wherein a compound inhibiting the binding of said rhinovirus strain or fragment thereof to said rhinovirus receptor protein or functional fragment thereof is present in step a).
3. The method of embodiment 2, wherein said compound is a molecule binding to said rhinovirus strain or fragment thereof and wherein said rhinovirus strain or fragment thereof is pre-incubated with said compound prior to step a).
4. The method of embodiment 2, wherein said compound is a molecule binding to said rhinovirus receptor protein or functional fragment thereof and said receptor protein is pre-incubated with said compound prior to step a).
5. A method for identifying a compound for the treatment or prevention of a rhinovirus infection comprising the steps of
   a) incubating a first solid support comprising at least one type of a rhinovirus receptor protein or a functional fragment thereof immobilized thereon with a rhinovirus strain or fragment thereof and a first amount of said compound;
   b) incubating a second solid support comprising at least one type of a rhinovirus receptor protein or a functional fragment thereof immobilized thereon with a rhinovirus strain or fragment thereof and a second amount of said compound, wherein said second amount of said compound is lower than the first amount of step a),
   c) determining the levels of said rhinovirus strain or fragment thereof binding to said rhinovirus receptor protein or functional fragment thereof in the presence of the first and the second amount of said compound;
   d) comparing the binding levels determined in step c), and
   e) identifying said compound as rhinovirus infection treating or preventing compound if the binding level in the presence of the first amount of said compound is lower compared to the binding level in the presence of the second amount of said compound.
6. The method of embodiment 5, wherein a detection molecule and/or a labelling molecule is used in step c) for determining said binding levels.
7. The method of any one of embodiments 1 to 6, wherein said detection molecule is an anti-rhinovirus antibody or functional fragment thereof, wherein said anti-rhinovirus antibody binds to said rhinovirus strain or fragment thereof outside the rhinovirus-receptor binding domain.
8. The method of any one of embodiments 1 to 7, wherein said labelling molecule is covalently linked to said rhinovirus strain or fragment thereof.
9. The method of embodiment 8, wherein said labelling molecule is biotin.
10. The method of any one of embodiments 1 to 9, wherein said rhinovirus strain is from rhinovirus A, rhinovirus B or rhinovirus C.
11. The method of any one of embodiments 1 to 10, wherein said fragment of a rhinovirus strain is selected from the group consisting of the capsid proteins VP1, VP2, VP3 and VP4, and any subfragment thereof.
12. The method of any one of embodiments 1 to 11, wherein said rhinovirus (RV) strain is selected from the group consisting of RV89, RV8, RV16, RV18, RV54, RV2, RV25, RV29, RV44, RV14 and RV72.
13. The method of any one of embodiments 1 or 12, wherein said at least one type of rhinovirus receptor protein is intercellular adhesion molecule 1 (ICAM-1), low density lipoprotein receptor (LDLR), cadherin-related family member 3 (CDHR3) or a functional fragment thereof, wherein said functional fragment comprises a binding moiety for a rhinovirus strain or fragment thereof.
14. The method of any one of embodiments 5 to 13, wherein said rhinovirus strain or fragment thereof is pre-incubated with said compound prior to step a).
15. The method of any one of embodiments 5 to 14, wherein said rhinovirus receptor protein or functional fragment thereof immobilized on said solid support is pre-incubated with said compound prior to step a).
16. The method of any one of embodiments 2 to 15, wherein said compound is a molecule binding to said rhinovirus strain or fragment thereof.
17. The method of any one of embodiments 2 to 16, wherein said compound is an antibody binding to said rhinovirus strain or fragment thereof.
18. The method of any one of embodiments 2 to 17, wherein said compound is a rhinovirus-neutralizing antibody.
19. The method of embodiment 18, wherein said rhinovirus-neutralizing antibody is an anti-VPl or anti-VP2 antibody, preferably a human or humanized anti-VPl antibody.
20. The method of any one of embodiments 2 to 16, wherein said compound is a rhinovirus receptor protein or functional fragment thereof, preferably a recombinant rhinovirus receptor protein or functional fragment thereof.
21. The method of any one of embodiments 2 to 15, wherein said compound is a molecule binding to said rhinovirus receptor protein or functional fragment thereof.
22. The method of any one of embodiments 2 to 15 or 21, wherein said compound is an antibody selected from the group consisting of anti-ICAM-1 antibodies, anti-LDLR antibodies and functional fragments thereof.
23. The method of embodiment 22, wherein said antibody is a human or humanized anti-ICAM-1 antibody.
24. The method of embodiment 22 or 23, wherein said antibody is an anti-ICAM-1 antibody directed against an N-terminal or C-terminal ICAM-1 peptide, wherein said N-terminal ICAM-1 peptide preferably consists of an amino acid sequence selected from the group consisting of SEQ ID No. 1, SEQ ID No. 2, SEQ ID No. 3 and SEQ ID No. 4, and wherein said C-terminal ICAM-1 peptide preferably consists of an amino acid sequence of SEQ ID No. 10.
25. The method of any one of embodiments 5 to 24, wherein a bioassay is performed, wherein said bioassay is preferably an enzyme-linked immunosorbent assay (ELISA), or an assay based on a solid support selected from the group consisting of sepharose beads and nitrocellulose.
26. A kit for identifying a compound for the treatment or prevention of rhinovirus infections comprising
   a) a solid support coated with at least one type of rhinovirus receptor protein or a functional fragment thereof;
   b) at least one rhinovirus strain or a fragment thereof; and
   c) at least one detection and/or labelling molecule.
27. The kit according to embodiment 26, wherein said at least one type of rhinovirus receptor protein is intercellular adhesion molecule 1 (ICAM-1), low density lipoprotein receptor (LDLR), cadherin-related family member 3 (CDHR3), or a functional fragment thereof.
28. The kit according to embodiment 26 or 27, wherein said at least one detection molecule is an anti-rhinovirus detection antibody, preferably combined with an enzyme-labelled secondary detection antibody, wherein said anti-rhinovirus detection antibody binds to said rhinovirus strain or fragment thereof outside the rhinovirus-receptor binding domain.
29. The kit according to embodiment 26 or 27, wherein said at least one labelling molecule is covalently linked to said rhinovirus strain or fragment thereof.
30. The kit according to embodiment 29, wherein said labelling molecule is biotin.
31. Use of a kit according to any one of embodiments 26 to 30 for identifying compounds for the treatment and prevention of rhinovirus infections.
32. Composition for the use in the treatment or prevention of viral diseases comprising at least one fragment derived from amino acid residues 28 to 110 and/or from amino acid residues 390 to 480 of ICAM-1, at least one variant of said at least one fragment or at least one antibody or functional fragment thereof binding to said at least one fragment, wherein said at least one fragment consists of 20 to 80 amino acid residues.
33. Composition for the use according to embodiment 32, wherein said at least one fragment or variant thereof is fused or conjugated to a carrier protein.
34. Composition for the use according to embodiment 32 or 33, wherein the at least one fragment derived from amino acid residues 28 to 110 of ICAM-1 comprises or consists of an amino acid sequence selected from the group consisting of SEQ ID No. 1, SEQ ID No. 2, SEQ ID No. 3 and SEQ ID No. 4.
35. Composition for the use according to any one of embodiments 32 to 34, wherein the at least one fragment derived from amino acid residues 390 to 480 of ICAM-1 comprises or consists of SEQ ID No. 10.
36. Composition for the use according to any one of embodiments 32 to 35, wherein said composition further comprises adjuvants and pharmaceutically acceptable excipients.
37. Compound comprising a peptide consisting of an amino acid sequence selected from the group consisting of SEQ ID No. 1, SEQ ID No. 2, SEQ ID No. 3, SEQ ID No. 4 and SEQ ID No. 10.
38. An antibody directed against a C-terminal peptide of ICAM-1 for use in the treatment or prevention of a rhinovirus infection.

### DESCRIPTION OF THE FIGURES

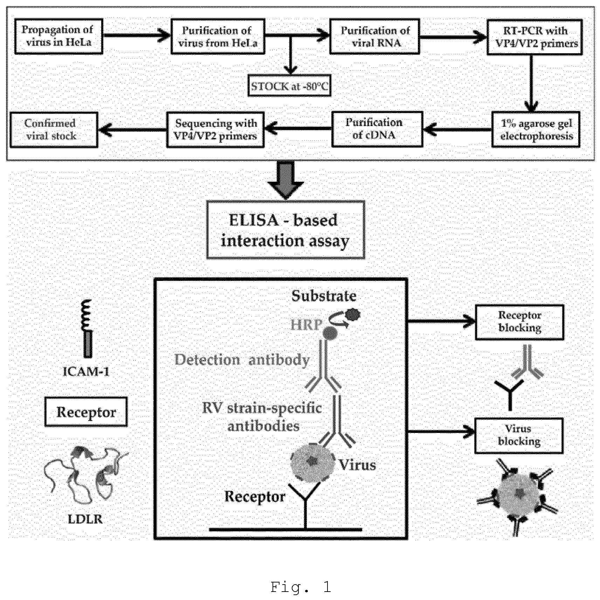

**Fig. 1** shows a flow chart summarizing the experimental design and reagents for the ELISA-based virus-receptor interaction assay. On top, the preparation of viral strains and the confirmation of their identity by RT-PCR are described. The scheme for the ELISA detection of virus-receptor interactions is shown in the lower part.

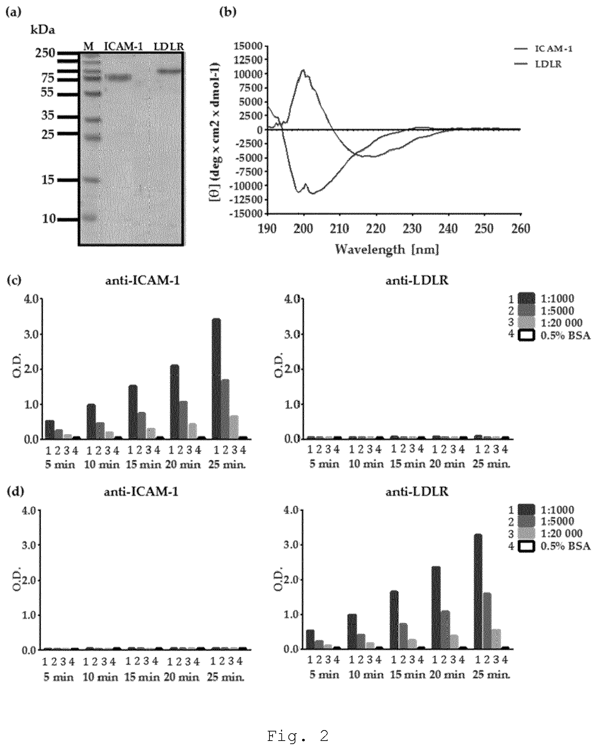

**Fig. 2** shows graphs regarding the characterization of recombinant human ICAM-1 and LDLR: (a) Coomassie-blue stained SDS-PAGE with recombinant human ICAM-1 (lane 1) and LDLR (lane 2). A molecular-weight marker (kDa: kilo Dalton) is indicated on the left. (b) Mean residue ellipticities (θ, y-axis) of ICAM-1 (red) and LDLR (blue) recorded at different wavelengths (nm, x-axis) by circular dichroism (CD) spectroscopy. Detection of (c) ICAM-1 or (d) LDLR with serial dilutions of anti-ICAM-1 antibodies (left), anti-LDLR antibodies (right) or BSA-containing buffer alone (BSA) by ELISA; shown are means of optical density (OD) values corresponding to bound antibodies (y-axes) measured at different time points (x-axes).

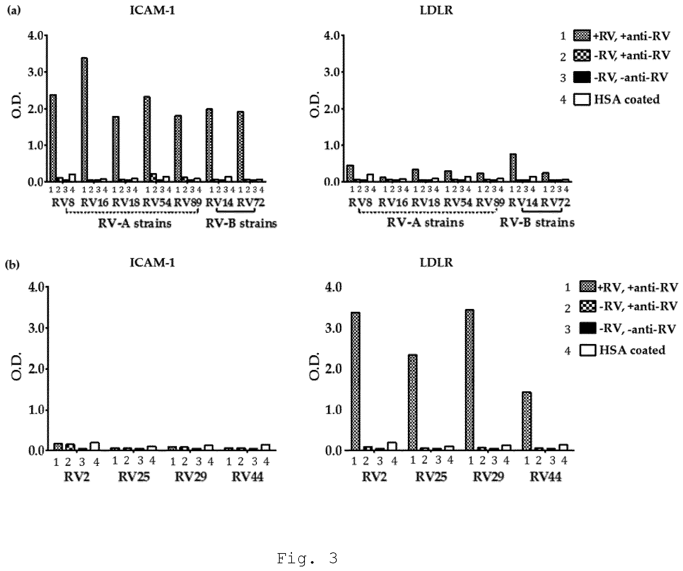

**Fig. 3** shows graphs regarding the binding of major group and minor group RVs to ICAM-1 and LDLR; shown are mean optical density (i.e., O.D.) values (y-axes) corresponding to receptor (ICAM-1 or LDLR)-bound (a) major or (b) minor group RVs (x-axes) as determined by ELISA. Tested major group RV-A or RV-B species (a) and minor group RV-A species (b) are indicated on the x-axes. The analyses were performed with virus (+50 µg/ml RV) and, for control purposes, without virus (-RV), without virus and detection antibodies (-RV, -anti-RV) or with I- instead of receptor-coated plates (I coated).

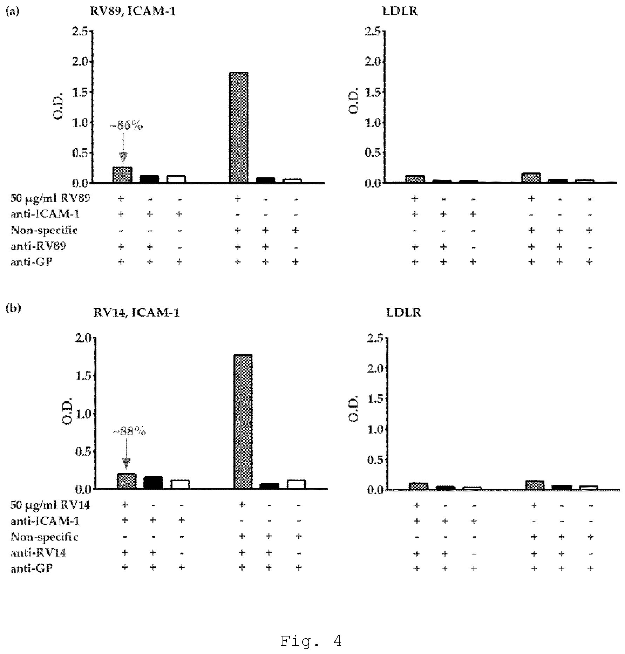

**Fig. 4** shows graphs regarding the inhibition of RV89 and RV14 binding to plate-bound receptors by receptor-specific antibodies. Shown is binding of RV89 (upper part) and RV14 (lower part) to plate-bound ICAM-1 (left panels) or plate-bound LDLR (right panels) in the form of mean optical density (i.e., O.D.) values (y-axes) in the presence (+) or absence (-) of virus, anti-ICAM-1 antibodies, non-specific antibodies, anti-RV detection antibodies and secondary detection antibodies (anti-GP) (see below x-axes). The percentage inhibitions of RV89 binding and RV14 binding obtained with anti-ICAM-1 antibodies versus non-specific antibodies are indicated.

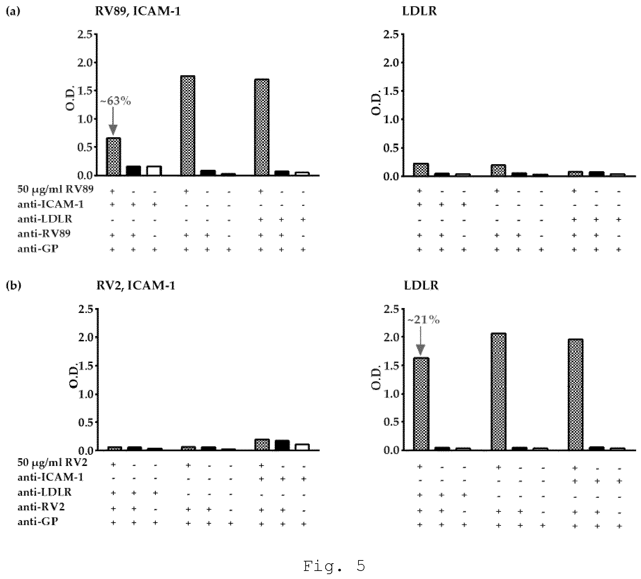

**Fig. 5** shows graphs regarding the inhibition of major group RV89 and minor group RV2 binding to plate-bound receptors by receptor-specific antibodies. Shown is binding of RV89 (upper part) and RV2 (lower part) to plate-bound ICAM-1 (left panels) or plate-bound LDLR (right panels) in the form of mean optical density (i.e., O.D.) values (y-axes) in the presence (+) or absence (-) of virus, commercial anti-ICAM-1 antibodies, commercial anti-LDLR antibodies, anti-RV detection antibodies and secondary detection antibodies (anti-GP) (see below x-axes). The percentage inhibitions of RV89 binding and RV2 binding obtained with commercial anti-receptor antibodies compared to without anti-receptor antibodies are indicated.

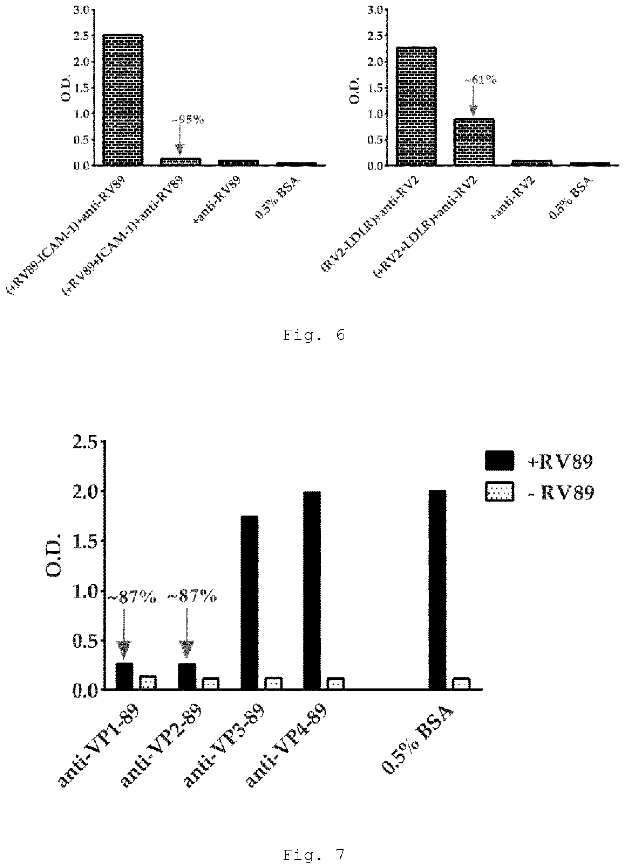

**Fig. 6** shows graphs regarding the auto-inhibition of binding of major group RV89 and minor group RV2 to their receptors. Shown is binding of RV89 to ICAM-1 (left part) and of RV2 to LDLR (right part) without (first column) or with (second column) pre-incubation with the receptor plus controls in which virus and receptor (third column) or virus, receptor and detection antibodies (fourth column) were omitted in the form of mean optical density (i.e., O.D.) values (y-axes). The percentage inhibitions of RV89 binding and RV2 binding obtained by pre-incubation with receptors are indicated.
**Fig. 7** shows a graph regarding the inhibition of RV89 binding to ICAM-1 by antibodies specific for each of the four viral capsid proteins (VP). Shown is binding of RV89 to ICAM-1 after pre-incubation of virus with antibodies against VP1, VP2, VP3 and VP4 as compared to pre-incubation with buffer containing BSA only in the form of mean optical density (i.e., O.D.) values (y-axes). The inhibitions of RV89 binding by anti-VPl and anti-VP2 antibodies are indicated by arrows. Omission of RV89 served as a negative control.

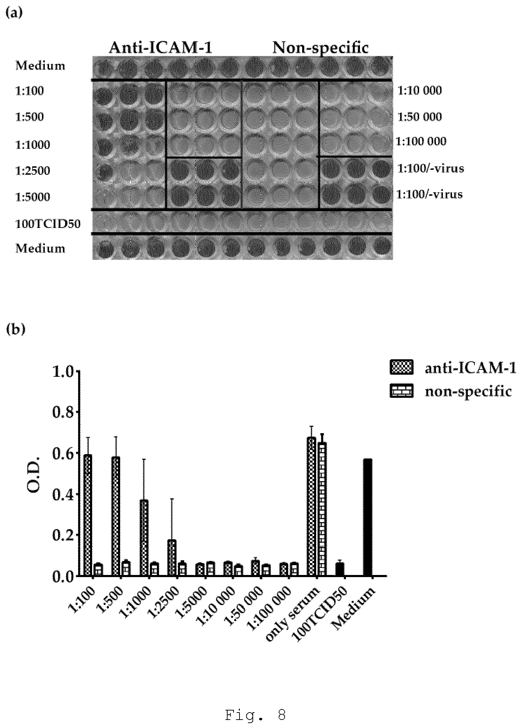

**Fig. 8** shows graphs regarding the inhibition of RV89 infection by anti-ICAM-1 antibodies determined by a cell-culture based virus inhibition assay. (a) Crystal Violet-stained viable cells incubated only with anti-ICAM-1 or non-specific antibodies (top), different dilutions of the antibodies (1:100-1:100.000) (anti-ICAM-1 and non-specific antibodies separated by red vertical line) in the presence of virus (100 TCID50), 1:100 diluted antibodies without virus, cell incubated only with virus (100 TCID50) or only with medium (bottom). (b) Mean OD values corresponding to viable cells in triplicate wells +/- standard deviations (SDs) (y-axis) of the above experiment (x-axis: conditions applied).

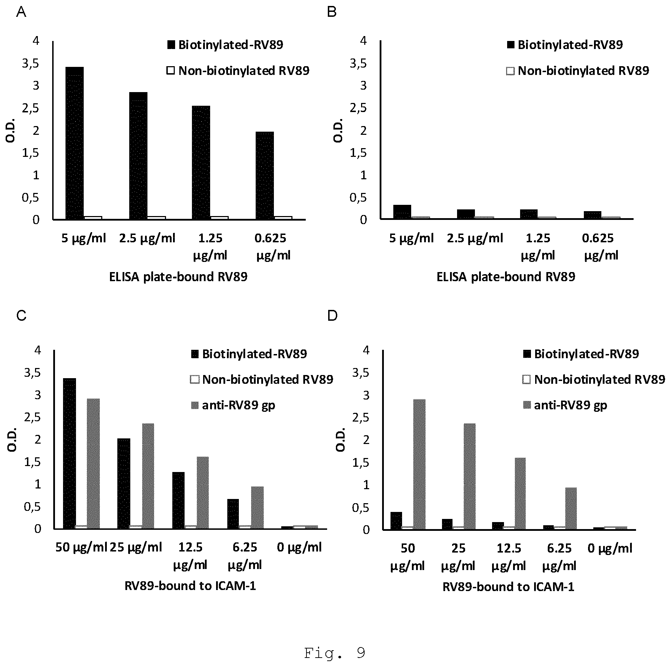

**Fig. 9** shows the detection of the biotinylated RV89 by Streptavidin-HRP and Avidin-HRP protein conjugates. (A, B) Displayed are optical density levels, O.D., (y-axes) corresponding to the ELISA plate-bound biotinylated or non-biotinylated RV89 (x-axes) detected either by Streptavidin-HRP (A) or Avidin-HRP (B). (C, D) Detection of RV89 specific binding to the ELISA-plate bound ICAM-1. Different amounts of biotinylated (black bars) or non-biotinylated (white bars) RV89 (x-axes) were detected either by Streptavidin-HRP (C) or Avidin-HRP (D). Grey bars represent the detection of non-biotinylated RV89 by anti-RV89 guinea pig antiserum followed by HRP-labelled sheep anti-guinea pig antibodies. Optical density values (i.e., O.D.: y-axes) were read at 405 nm.
**Fig. 10** shows the human ICAM-1 sequence (UniProt accession number: P05362), the peptides P1-P10 and the domains of the human ICAM-1 structure.

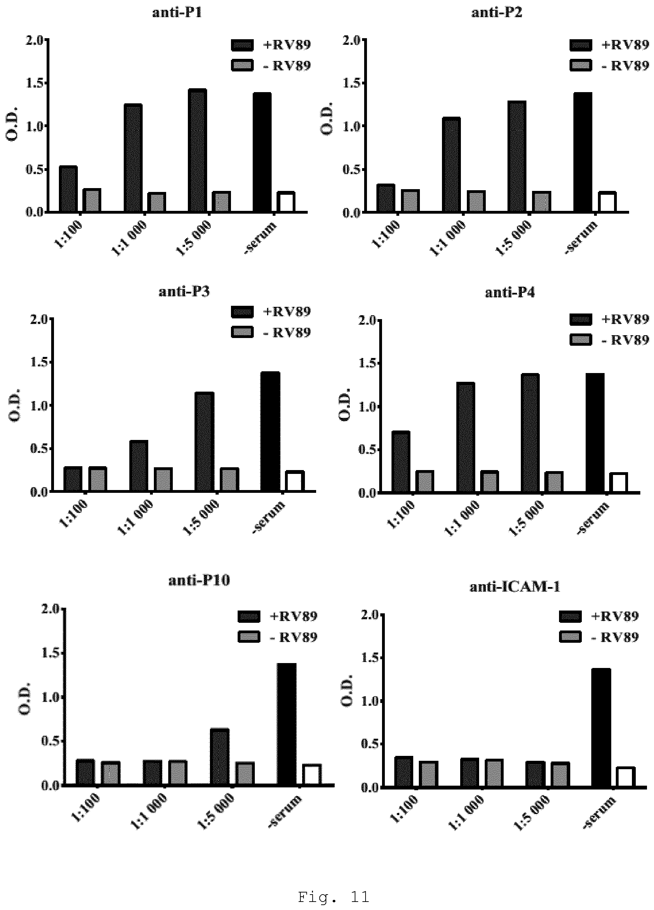

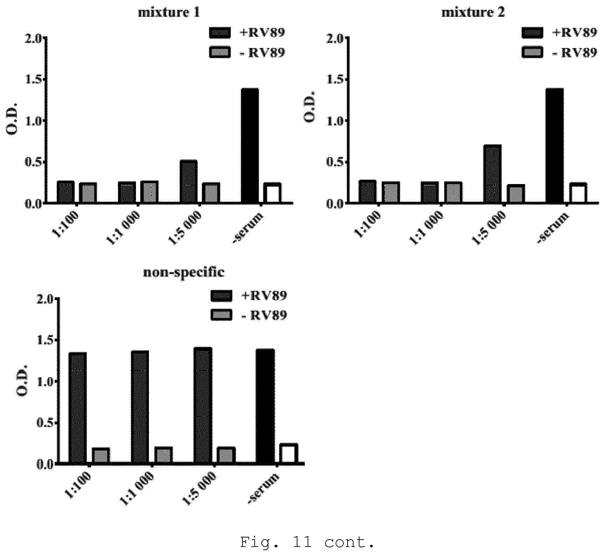

**Fig. 11** shows the inhibition of RV89 binding to immobilized ICAM-1 by ICAM-1- and ICAM-1 peptide-specific antibodies. Shown is binding of RV89 to plate-bound ICAM-1 in the form of mean optical density (i.e., O.D.) values (y-axes) in the presence of different dilutions of ICAM-1 peptide-specific antibodies (anti-P1, anti-P2, anti-P3, anti-P4, anti-P10, mix 1 containing anti-P1, P2, P3, P4, P10, mix 2 containing anti-P1, P2, P3, P4), anti-ICAM-1 antibodies, normal antibodies or without any added antibodies (-serum) (x-axes). White columns show control experiments without addition of RV89.

### EXAMPLES

### Example 1: Materials and General Methods

### Propagation of RV strains

The H1 HeLa cell line (American Type Culture Collection ATCC, USA) was cultured in Minimum Essential Medium (MEM) containing Earle's salt (Gibco, Thermo Fisher Scientific, USA), supplemented with 10% fetal bovine serum (FBS) (Gibco) and antibiotics (Penicilin-Streptomycin (10.000 U/mL), 60 µl Gentamycin (1.2 µg/ml, Gibco)). Cells were grown in an incubator Cytoprem 2 CO2 (Thermo Fisher Scientific, USA) at 37°C, 5% CO2 and 91% relative humidity. Human rhinovirus strains (Major group RV-A: RV89, RV8, RV16, RV18, RV54; Minor group RV-A: RV2, RV25, RV29, RV44; Major group RV-B: RV14, RV72) obtained from ATCC were added to MEM medium supplemented with 1% FBS, 15 mM Magnesium chloride (Sigma-Aldrich, Merck KGaA, Germany) without antibiotics (Infectious medium) and used to infect HeLa cells which had been grown in a 25 cm² cell culture flask with canted neck and screwed cap (TPP Techno Plastic Products AG, Switzerland) until they had reached 70-80% confluency. Then cells were incubated for 2 h at 34°C, 5% CO₂ and 91% relative humidity, washed with Dulbecco's Phosphate-Buffered Saline (DPBS) (Gibco) and incubated in Infectious medium for the following 1-7 days depending on the strain being propagated. When the cytopathic effect (CPE) was observed, dead cells were centrifuged 5 min at 1200 rpm (210 x g, Rotanta 46RC, Germany). The supernatant was used for the infection of HeLa cells, which had been grown to confluency in a 150 cm² cell culture flask with canted neck and vented cap (Corning, USA) following the same procedure. Finally, HeLa cells which had been grown to confluence in 10 cell culture flasks of 150 cm² size were infected to obtain sufficient amounts of virus.

### Purification of RV strains

Dead cells were centrifuged for 20 min at 1200 rpm (210 x g, Rotanta 46RC, Hettich). This first supernatant was collected and the pellet was suspended in 10 ml cell lysis buffer (pH 7.4) containing 0.38% (w/v) ethylenediaminetetraacetic acid (EDTA) (BIOMOL, Germany) and 0.12% (w/v) NaCl (Merck-Millipore, Merck, Germany). The suspension was subjected to three freeze-thaw cycles performed in liquid nitrogen and cold water, respectively and further homogenized using a Dounce Homogenizer (Carl Roth, Germany). This homogenate was centrifuged for 20 min at 4000 rpm (2330 x g, Rotanta 46RC, Hettich). The second supernatant was then combined with the first and polyethylenglycol 6000 (PEG6000) (7% w/v) (Merck-Millipore, Merck, Germany) and NaCl (3% w/v) were added. The virus-containing solution was incubated by moderate shaking overnight at 4°C and was centrifuged for 20 min at 4°C, 4000 rpm (2330 x g, Rotanta 46RC, Hettich). The viral pellets were suspended in 10 ml DPBS and by vortexing for 1 h at room temperature. The supernatant containing purified virus preparations was after the last centrifugation for 5 min at 4°C, 12.700 rpm (18 213 x g, Eppendorf 5427R, Germany) aliquoted and stored at -80°C (200 µl/stock). The protein contents of purified virus preparations were determined by the Bicinchoninic acid assay (BCA), Protein Assay Kit (Thermo Fisher Scientific).

### Analysis of virus preparations

Viral RNA was extracted using a QIAmp Viral RNA Kit (Qiagen GmbH, Germany). Viral RNA was transcribed into cDNA and amplified by one-step RT-PCR using SuperScript III First Strand Synthesis System SuperMix (Invitrogen, USA) with VP4/VP2 forward (5'-GGG ACC TAC TTT GGG TGT CCG TGT-3'; SEQ ID No. 13) and reversed (5'-GCA TCI GGY ARY TTC CAC CAC CAN CC-3'; SEQ ID No. 14) primers (Eurofins Genomics, Ebersberg, Hamburg, Germany), where I = inosine; Y = C, T; R = A, G; N = A, G, C, T. RT-PCR was done in a thermal cycler MWG Primus 96 plus PCR (Stratagene, Germany) by performing reverse transcription for 1 h at 50°C followed by 20 min of denaturation at 94°C and 40 cycles of amplification each comprising 1 min denaturation at 94°C, 1 min annealing at 55°C and 1 min elongation at 72°C. A final extension was done for 10 min at 72°C. The amplicons in a total volume of 50 µl were mixed with 6x DNA Loading Dye (Thermo Fisher Scientific) and subjected to 1% agarose gel electrophoresis (Biozym LE Agarose, Biozym Scientific GmbH, Germany) using ethidium bromide for staining of DNA (Ethidium bromide solution, Sigma-Aldrich, Merck, Germany). A DNA Ladder GeneRuler 1kb Plus DNA Ladder (Thermo Fisher Scientific) was used as marker. PCR products were extracted from the gel and purified with Wizard^{®} SV Gel and PCR Clean-Up System (Promega Corporation, Madison, WI, USA) and used for cycle sequencing on an ABI 3730XL sequencer with the same forward and reversed primers which had been used for amplification Eurofins Genomics (Germany).

Forward and reversed sequences were aligned using the program BLASTn (https://blast.ncbi.nlm.nih.gov/Blast/) to identify unambiguous double strand sequences, which were compared with the sequences deposited in the database of the National Center for Biotechnology (NCBI) using web search engine BLASTn. These sequences were translated into protein sequences using the ExPASy translation tool (https://www.expasy.org/), and subjected to multiple sequence alignment by Clustalθ with ClustalW as an output format (https://www.ebi.ac.uk/Tools/msa/clustalo/). Editing was performed with the GeneDoc 2.7 software (https://genedoc.software.informer.com/2.7/). Conserved regions along with conservative, semi-conservative and non-conservative exchanges were visualized in Microsoft Word 2010 and Adobe Illustrator CS (64 Bit).

Evolutionary analysis of protein sequences was performed using the Maximum likelihood (ML) method implemented in the Molecular Evolutionary Genetics Analysis (MEGA) software version X (64 bit) (https://www.megasoftware.net). As a statistical method, Bootstrap method with 1000 repeats was applied. Before the generation of phylogenetic tree, a substitution model with the lowest Bayesian Information Criterion (BIC) and Akaike Information Criterion, corrected (AICc) were estimated by the Find Best Protein Models tool implemented in MEGA-X. As the result, general matrix (LG) model (Le and Gascuel, Mol. Biol. Evol. 25 (7): 1307-20 (2008)) was chosen. The tree was further rearranged with the Nearest-Neighbor-Interchange (NNI) option.

A phylogenetic tree was constructed from the 11 representative major group and minor group RV strains. All positions with less than 95% site coverage were eliminated from the analysis (i.e., fewer than 5% alignment gaps were allowed at any position; partial deletion option).

### Receptor- and virus-specific antibodies

Specific rabbit antibodies were obtained by immunizing rabbits with 200 µg aliquots of recombinant ICAM-1 (R&D Systems), purified recombinant VP2, VP3 and VP4, which had been purified as described (Niespodziana et al., The FASEB J. 26 (3): 1001-1008 (2012)). The first immunizations were carried out with a mix of the respective protein and CFA and the 2nd to 4th immunization with a mix of each protein and IFA (Charles River, France). Rabbit anti-VPl antibodies are described (Edlmayr et al., J. Immunol. 182 (10): 6298-306 (2009)). Commercial rabbit anti-ICAM-1 (Sino Biological Beijing, China) and goat anti-LDLR antibodies (R&D Systems) used in certain experiments were purchased. Normal rabbit antibodies used for control experiments were obtained from Genscript (Netherlands). RV strain-specific guinea pig antibodies were purchased from ATCC.

### Example 2: Development of an ELISA-based RV-receptor interaction assay

A simple ELISA-based assay was built which allows to investigate the interaction of RV strains with their corresponding receptors using defined components (Fig. 1). Viral stocks from a panel of major group RV-A strains (RV89, RV8, RV16, RV18, RV54) and major group RV-B strains (RV14, RV72) which use ICAM-1 as receptor as well as minor group RV-A strains (RV2, RV25, RV29, RV44) which bind to LDLR were established, purified and analysed as described in Example 1. The ELISA-based assay was constructed in order to allow purified virus to interact with ELISA plate-bound receptor molecules. For this purpose, purified ICAM-1 and LDLR molecules were obtained and analysed as described below.

### Analysis of recombinant human receptor proteins

Recombinant human ICAM-1/CD54 and LDLR were obtained from R&D Systems (USA). The purity and molecular weight of ICAM-1 and LDLR were determined by SDS-PAGE gel under reducing conditions and subsequent Coomassie-blue staining of the gel. A protein ladder PageRulerTM (Thermo Fisher Scientific) was used as molecular weight marker. Recombinant glycosylated ICAM-1 migrated as band of approximately 80 kDa and LDLR appeared as band of 120-140 kDa (Fig. 2a).

Secondary structure of the proteins was studied by circular dichroism (CD) spectroscopy in the far ultraviolet (UV) wavelength region between 260 and 190 nm using a Jasco J-810 spectropolarimeter (Japan Spectroscopic Co., Japan). Samples dissolved at a concentration of 0.1 mg/ml in PBS were analyzed in 0.2 cm path-length cuvettes (Hellma Analytics) at 20°C as described in Gallerano et al., Amino Acids 40 (3) : 981-9 (2011)). The spectrum of ICAM-1 was characterized by a maximum at 200 nm and two minima at 216 and 219 nm indicating that the protein is folded and assumes *β*-sheet secondary structure typical for Ig-like domains (Fig. 2b). The result from the secondary structure fitting procedure estimated 46% *β*-sheets, 3% α-helices, 20% turns and 29% unordered structure. Recombinant LDLR seems to be mainly unfolded, characterized by a maximum at 193 nm and two minima, the local at 199 nm and the global one at 203 nm (Fig. 2b). Secondary structure fitting estimated a limited amount of α-helices (11%) and *β-*sheets (39%).

In addition, an immunological characterization of ICAM-1 and LDLR was performed by testing the proteins for reactivity with rabbit anti-ICAM-1 antibodies (Sino Biological Beijing, China) and goat anti-LDLR antibodies from R&D Systems. Proteins were coated to 96-well half area microplates (Greiner Bio-One International GmbH, Kremsmünster, Austria) (2 µg/ml) overnight at 4°C, washed twice with phosphate buffer saline containing 0.05% (v/v) Tween 20 (PBS-T), and blocked with 2% (w/v) bovine serum albumin (BSA). Bound rabbit antibodies were detected with 1:2000 diluted horseradish peroxidase (HRP) labelled donkey anti-rabbit antibodies (Amersham, Malborough, MA) and bound goat antibodies were detected with rabbit HRP-labelled anti-goat antibodies (R&D Systems). Fig. 2c shows that ICAM-1 reacted with anti-ICAM-1 but not anti-LDLR antibodies in a concentration-dependent manner. LDLR specifically and dose-dependently reacted with LDLR-specific but not ICAM-1-specific antibodies (Fig. 2d).

### ELISA-based virus-receptor interaction assay

Half area 96-well microplates (Greiner Bio-One International GmbH, Austria) were coated with 2 µg/ml of ICAM-1, LDLR and human serum albumin (HSA) (negative control) overnight at 4°C as described (Stenberg-Hammar et al., Allergy 71 (12): 1728-1735 (2016)). Plates were washed twice with phosphate buffer saline containing 0.05% (v/v) Tween 20 (PBS-T), blocked with 2% (w/v) bovine serum albumin (BSA) and incubated for 4 h at 24°C. Plates were then incubated with major group or minor group RV strains (50 µg/ml according to the protein determination of the viral stock) overnight at 4°C. Receptor-bound virus was then detected with strain-specific guinea pig antibodies (ATCC; 1:5000) followed by HRP-labelled sheep anti-guinea pig antibodies (Jackson IR, Cambridgeshire, UK). The colour reaction was developed with substrate solution containing ABTS and H2O2 as described (Stenberg-Hammar et al., Allergy 71 (12): 1728-1735 (2016)). The mean optical density (O.D.) values corresponding to the amount of receptor-bound virus were measured at 405 nm and 492 nm (reference) in a TECAN Infinite F5 ELISA reader with the integrated software i-control 2.0 (Tecan Group Ltd., Switzerland). Measurements were performed in duplicate wells deviations of less than 10% between duplicates.

Fig. 3a shows that major group RV-A as well as distantly related RV-B strains were able to bind specifically to the plate bound ICAM-1. No binding was detected when plates were coated with the control protein HSA and when virus or virus and anti-RV antibodies were omitted (Fig. 3a, left panel). No binding of minor group RV strains was detected to ICAM-1 confirming the specific interaction of major group RV strains to recombinant ICAM-1 (Fig. 3b, left panel). However, minor group RV strains bound specifically to ELISA plate-bound LDLR but not to the control protein, HSA (Fig. 3b, right panel). There was also no binding when virus or virus and RV-specific detection antibodies were omitted. Some weak binding of major group RV strains (i.e., RV8, RV18, RV54, RV89, RV14 and RV72) to LDLR was observed above signals obtained with the control protein HSA (Fig. 3a, right panel).

### Example 3: ELISA-based inhibition of virus-receptor interaction

The ELISA-based RV-receptor interaction assay allows investigating components either specific for the receptor or specific for the virus regarding their capacity to inhibit the virus-receptor interaction. For this purpose, receptor proteins were coated to the ELISA plates and incubated with the RV strains. The binding of RV strains could be detected with RV strain-specific antibodies and a HRP-labelled secondary antibody followed by substrate and colour reaction (Fig. 1). In this setting, plate-bound receptor can be pre-incubated with receptor-specific compounds or virus can be pre-incubated with virus-specific agents (Fig. 1, bottom).

In all inhibition assays ELISA microplates were coated with recombinant human ICAM-1 and LDLR (2 µg/ml), washed and blocked, as described in Example 2. In blocking experiments performed with the rabbit-anti-ICAM-1 antibodies, rabbit sera or for control purposes, sera from normal rabbits were added in different dilutions (1:10-1:100.000). Commercial anti-ICAM-1 and anti-LDLR antibodies were diluted to a concentration of 10 µg/ml for blocking experiments. After washing of plates, virus preparations were allowed to bind and detected.

In fluid phase competitions assays, RV89 (25 µg/ml) and RV2 (2 µg/ml) was pre-incubated in solution with their corresponding receptors (50 µg/ml) overnight at 4°C before they were allowed to bind to plate-bound receptors.

The effects of antibodies specific for RV89-derived coat proteins were also studied by fluid phase inhibition. For this purpose, RV89 (25 µg/ml) was pre-incubated with each anti-VP antiserum diluted 1:5 or, for control purposes, only with buffer containing 0.5% BSA before virus was added to plate bound receptors. The detection of bound RV strains in the fluid phase inhibitions was performed as described in Example 2. The percentages inhibitions of receptor-bound virus were calculated as follows: Percentage inhibition of RV binding = 100 - ODⁱ/ODⁿ x 100, where ODⁱ and ODⁿ represent the mean extinctions obtained with inhibitors versus without inhibitor, respectively. All figures were generated in GraphPad Prism 6 and Adobe Illustrator CS (64 Bit).

### Inhibition of RV binding to their receptors with polyclonal antibodies raised against ICAM-1 and commercial anti-ICAM-1 and anti-LDLR antibodies

In addition to the commercial ICAM-1-specific antibodies, an antiserum specific for ICAM-1 was raised by immunizing a rabbit with recombinant ICAM-1 and tested regarding its capacity to inhibit binding of two distantly related major group RV strains (i.e., RV89, RV14) to ICAM-1 (Fig. 4). It was found, that the anti-ICAM-1 antibodies but not normal rabbit antibodies almost completely (i.e., >85% inhibition) blocked binding of RV89 as well as of RV14 to plate-bound ICAM-1 (Fig. 4a, b, left panels). The rabbit anti-ICAM-1 antibodies were highly potent in blocking the RV-ICAM-1 interaction, because they still reduced the binding of RV89 to ICAM-1 even after a dilution up to 1:100.000, whereas normal rabbit antibodies had no effect on RV-receptor binding. A very weak but discrete binding of RV89 and RV14 to LDLR was observed in the control experiment performed with plate-bound LDLR (Fig. 4a, b, right panels).

In a next set of experiments, the commercial anti-ICAM-1 and anti-LDLR antibodies (see Example 1) were tested for their ability to inhibit the binding of major group RV89 and minor group RV2 to ICAM-1 and LDLR in the ELISA-based virus-receptor interaction assay (Fig. 5). It was found that, at given dilution, the commercial anti-ICAM-1 antibodies inhibited RV89 binding to ICAM-1 to a lower degree (i.e., approx. 63% inhibition) than the antibodies against recombinant ICAM-1 (i.e., >85% inhibition) (Fig. 5a, Fig. 4a). Anti-LDLR antibodies inhibited specifically minor group RV2 binding to LDLR whereas anti-ICAM-1 antibodies had no inhibitory activity. However, at the used dilution, the inhibition by anti-LDLR antibodies was only approximately 21% (Fig. 5b). Some very weak binding of RV89 to LDLR was observed which appeared reduced upon pre-incubation of LDLR with anti-LDLR antibodies (Fig. 5a, right panel) . No binding of minor group RV2 to ICAM-1 was seen (Fig. 5b, left panel).

### Inhibition of RV binding to their receptors with soluble recombinant receptors

It was tested if soluble recombinant receptors can be used to block major and minor RV binding to the immobilized receptors and what concentration of soluble receptors may be needed for inhibition. The left panel of Fig. 6 shows that pre-incubation of RV89 with soluble ICAM-1 almost completely inhibited the binding of the virus to ICAM-1. The more than 95% inhibition of RV89 binding to ICAM-1was achieved with 50 µg/ml ICAM-1 and a concentration of 25 µg/ml of purified RV89. A more than 60% inhibition of minor group RV2 to LDLR was observed when virus was pre-incubated with soluble LDLR (Fig. 6, right panel) . The inhibition of RV2 binding to LDLR was obtained with 5 µg/ml RV2 in the presence of 50 µg/ml LDLR. The specificity of the assays was ensured by several controls, including pre-incubation omission of virus and receptor, omission of virus or omission of receptor and detection antibodies (Fig. 6).

### Identification of RV-neutralizing antibodies using the ELISA-based RV-receptor interaction assay

It was tested if the ELISA-based RV-receptor interaction assay can be used to screen RV-specific antibodies for their ability to block the RV-receptor interaction. RV89 was pre-incubated with rabbit antibodies which had been raised against the RV89 coat proteins VP1(Edlmayr et al., J. Immunol. 182 (10): 6298-306 (2009)), VP2, VP3 or VP4. For control purposes, performed pre-incubation was performed only with buffer containing 0.5% BSA. Interestingly, it was found that anti-VP1 and anti-VP2, but not anti-VP3 or anti-VP4 antibodies inhibited the binding of RV89 to ICAM-1 (Fig. 7). The inhibition obtained with anti-VPl and anti-VP2 antibodies was very strong (i.e., approximately 87%), indicating that the antisera contain virus-neutralizing antibodies. Indeed, the anti-VP1 antiserum has already been shown to exhibit virus-neutralizing activity in earlier cell culture-based virus neutralization assays (Edlmayr et al., J. Immunol. 182 (10): 6298-306 (2009), Edlmayr et al., Eur. Respir. J. 37 (1): 44-52 (2011)).

### Example 4: Comparison of ELISA-based RV-interaction assay with cell culture-based virus neutralization assay

### The ELISA-based RV-receptor interaction is more sensitive in showing anti-ICAM-1 effects than an established cell culture-based virus neutralization assay

The ELISA-based RV-interaction assay was compared with a traditional cell culture-based virus neutralization assay regarding the abilities of the assay to reveal the capacity of anti-ICAM-1 antibodies to inhibit the binding of RV89 to ICAM-1. Both analyses were performed with the same virus preparation and antibody dilutions. In cell culture-based virus-neutralization assay, HeLa cells were incubated for 3 h with serial dilutions of anti-ICAM-1 antibodies before they were infected with 100 TCID50/cell of RV89. Anti-ICAM-1 antibodies specifically inhibited RV89 infection up to a dilution of the antibodies of 1:2500 whereas no inhibition of infection was obtained with the normal rabbit antibodies at any dilution (Fig. 5a, b). Using the ELISA-based RV-interaction assay an inhibitory effect of anti-ICAM-1 antibodies on the virus receptor interaction could be detected up to a dilution of 1:50.000). Thus, the ELISA-based RV-interaction assay appeared to be 20-fold more sensitive in revealing the inhibitory effects of the anti-ICAM-1 antibodies. Further, the results for higher dilutions of the antibodies appear to be more consistent. The differences in duplicate determinations in the ELISA were always less than 10% whereas rather large SDs were noted in the cell culture-based virus-neutralization assay (Fig. 8).

### Example 5: Detection of the RV89 specific binding to ICAM-1 by biotinylation approach

In order to improve the detection of the specific binding of the virus to its corresponding receptor, intact RV89 was labelled with biotin (i.e., biotinylated) and two protein conjugates, Streptavididin-HRP and Avidin-HRP, were tested for the maximum detection. For the labelling of 1 ml of RV89 (0.25 mg/ml), 50 µl of EZ-Link NHS-PEG12-Biotin (1 mg/ml, Thermo Scientific, USA) were used and the mixture was incubated for 2h at RT. To get rid of the excess of the biotinylation reagent, 1 ml of the sample were applied to the 2 ml ZebaTM Spin Desalting Column (Thermo Scientific) previously equilibrated with 7.4 pH phosphate-buffered saline (PBS) and the column was centrifuged for 2 minutes at 1000 x g. The biotinylated virus was then collected, aliquoted and stored at -80°C until use. In order to test the efficiency of the biotinylation process, first an ELISA experiment was performed, in which ELISA plate was directly coated with different concentrations of the biotinylated and non-biotinylated RV89 (5 - 0.625 µg/ml in carbonate buffer). For the detection of RV89, two protein conjugates were tested, the ultrasensitive Streptavidin-Peroxidase Polymer (Merck, Darmstadt, Germany) and Avidin-HRP (BD Pharmingen, USA); both were diluted 1:1000 and incubated for 1h at RT as recommended by the manufacturer. Optical density (i.e., O.D.) values were then measured at 405 and 492 nm (Fig. 9A and 9B). As demonstrated in Fig. 9, RV89 could be detected by Streptavidin-HRP up to a concentration of 0.625 µg/ml, while the detection of the virus using the Avidin-HRP was less efficient. No binding was observed to the non-biotinylated RV89, which was used as a negative control (Fig. 9). Furthermore, to investigate whether the biotinylation could eventually influence the binding of the intact RV89 to the ICAM-1, another ELISA experiment was performed, in which first the viral receptor, ICAM-1, was coated onto the ELISA plate (1 µg/ml in carbonate buffer). After blocking with 2% BSA in PBS/T for 4 hours at RT, different amounts of either biotinylated or non-biotinylated RV89 were added in a concentration dependent manner (50 - 6.25 µg/ml RV89) and the plate was incubated again overnight at 4°C. For the detection of specific RV89 binding to ICAM-1, again the two protein conjugates were used, the Avidin-HRP and the ultrasensitive Streptavidin-Peroxidase Polymer diluted 1:1000 (Fig. 9C and 9D). In addition, anti-RV89 guinea pig antibodies diluted 1:5000 (ATCC, USA) followed by HRP-labelled sheep anti-guinea pig antibodies (1:1000) were used for comparison (Fig. 9C and 9D). As shown in Fig. 9, specific RV89 binding to the ELISA-plate bound ICAM-1 was similarly detected by both Streptavidin-HRP and anti-RV89 gp antibodies up to a concentration of 6.25 µg/ml. The detection of the virus-receptor interaction using the Avidin-HRP was much weaker. Again, no binding was observed to non-biotinylated RV89, which was used as a negative control. Importantly, it seems that the biotinylation process did not influence the binding of RV89 to ICAM-1. Thus, these findings show that it is generally possible to biotinylate any intact virus, which can subsequently be used in surrogate ELISA-based inhibition assays. This should improve significantly the assay's efficacy through the increase of the specificity of the detection system and the reduction of background signals.

### Example 6: Inhibition of RV89 binding to immobilized ICAM-1 by ICAM-1- and ICAM-1 peptide-specific antibodies

In order to investigate, whether antibodies directed to certain parts of human ICAM-1 are capable to inhibit the binding of RV89 to its cellular receptor, 11 peptides (P1 (SEQ ID No. 1), P2 (SEQ ID No. 2), P3 (SEQ ID No. 3), P4 (SEQ ID No. 4), P5 (SEQ ID No. 5), P6 (SEQ ID No. 6), P7 (SEQ ID No. 7), P8 (SEQ ID No. 8), P9 (SEQ ID No. 9), P10 (SEQ ID No. 10) and P11 (SEQ ID No. 11)) were synthesized that are part of the extracellular domain of human ICAM-1 (UniProt accession number: P05362, www.uniprot.org/uniprot/P05362) (Fig. 10). Peptides were synthesized using Fmoc (9 fluorenyl methoxy carbonyl) chemistry to protect amino acids (Merck Chemicals and Life Science GmbH, Germany) and Oxyma/DIC(N,N'-Diisopropylcarbodiimide, Merck) for the peptide coupling. Preloaded Wang resins were used as a solid phase. After synthesis, peptides were cleaved from the resins and purified via reversed phase HPLC (DionexUltiMate 3000, Thermo Fisher Scientific, Waltham, Massachusetts, United States) using an Aeris PEPTIDE XB-C18 LC Column (5 µm, 250 x 21.2 mm, AXIA^{™} Packed, Phenomenex Aschaffenburg, Germany OR Torrance, United States). The purity as well as the molecular masses of the peptides was confirmed by MALDI-TOF analyses (Bruker Daltonicsmicroflex, United States). Table 1 shows the amino acid sequences of the peptides and positions within the human ICAM-1 protein primary structure (see Fig. 10).

**Table 1: Characteristics of human ICAM-1-derived synthetic peptides. "Position aa" refers to the position of the peptides within the human ICAM-1 protein primary structure**

| **Peptide** | **SEQ ID No.** | **Sequence** | **Position aa** |
|---|---|---|---|
| P1 | 1 | QTSVSPSKVILPRGGSVLVTCSTSCDQPKLL | 28-58 |
| P2 | 2 | STSCDQPKLLGIETPLPKKELLLPGNNRKVYE | 49-80 |
| P3 | 3 | KKELLLPGNNRKVYELSNVQEDSQPMCYSNCPDG | 66-99 |
| P4 | 4 | YELSNVQEDSQPMCYSNCPDGQSTAKTFLTVY | 79-110 |
| P5 | 5 | WTPERVELAPLPSWQPVGKNLTLRCQVEGGA | 111-141 |
| P6 | 6 | LVRRDHHGANFSCRTELDLRPQGLELFENTSAPYQ | 174-208 |
| P7 | 7 | ALGDQRLNPTVTYGNDSFSAKASVSVTAEDEGTQRLTC | 253-290 |
| P8 | 8 | EVSEGTEVTVKCEAHPRAKVTLNGVPAQPLG | 320-351 |
| P9 | 9 | ATPEDNGRSFSCSATLEVAGQLIHKNQTRELRVL | 359-393 |
| P10 | 10 | GPRLDERDCPGNWTWPENSQQTPMCQAWGNPLPELKC | 395-431 |
| P11 | 11 | VTVTRDLEGTYLCRARSTQGEVTRKVTVNVLS | 445-476 |

Next, purified synthetic peptides were coupled to Keyhole limpet hemocyanin (KLH) and used for the immunization of rabbits. Induced ICAM-1 peptide-specific antibodies were then tested for their ability to inhibit the binding of RV89 to human ICAM-1 molecule by the surrogate ELISA-based inhibition assay. For this purpose, ELISA plates were coated with ICAM-1 (2 µg/ml, R&D Systems, Canada), blocked with 2% BSA and incubated with different dilutions of the different rabbit antisera (Fig. 11). After overnight incubation, 25 µg/ml of RV89 were added to the plates and the specific binding was detected by anti-RV89 guinea pig antibodies (1:5000, ATCC, USA) followed by 1:1000 HRP-labeled sheep anti-guinea pig antibodies (Jackson ImmunoResearch, UK). The optical density (OD) values corresponding to the level of the native virus bound to its receptor were measured at 405-495 nm in an ELISA reader (Infinite F50, Tecan, Switzerland). The presence of RV binding sites on the N-terminus of ICAM-1 has been reported earlier (McClelland et al., Proc. Natl. Acad. Sci. U S A. 88(18):7993-7. (1991)), but the C-terminal portion was not expected to play a role in rhinovirus binding. Surprisingly, as shown in Fig. 11, the highest inhibition of the RV89 binding to the immobilized ICAM-1 was obtained by antibodies directed against peptide 10 which was derived from the C-terminus of ICAM-1 (up to 1:500). Antibodies induced by the complete human ICAM-1 inhibited the RV89 binding up to a dilution of 1:5000 and were used as a positive control in the experiment. No inhibition of the binding was observed when normal rabbit serum was used.

## Claims

1. A method for determining the binding of a rhinovirus strain or fragment thereof to a rhinovirus receptor protein or functional fragment thereof comprising the steps of
a) incubating a solid support comprising at least one type of rhinovirus receptor protein or a functional fragment thereof immobilized thereon with a rhinovirus strain or fragment thereof, and
b) determining the binding level of said rhinovirus strain or fragment thereof to said rhinovirus receptor protein or functional fragment thereof,
wherein a detection molecule and/or a labelling molecule is used in step b) for determining said binding level.

2. A method for identifying a compound for the treatment or prevention of a rhinovirus infection comprising the steps of
a) incubating a first solid support comprising at least one type of a rhinovirus receptor protein or a functional fragment thereof immobilized thereon with a rhinovirus strain or fragment thereof and a first amount of said compound;
b) incubating a second solid support comprising at least one type of a rhinovirus receptor protein or a functional fragment thereof immobilized thereon with a rhinovirus strain or fragment thereof and a second amount of said compound, wherein said second amount of said compound is lower than the first amount of step a),
c) determining the levels of said rhinovirus strain or fragment thereof binding to said rhinovirus receptor protein or functional fragment thereof in the presence of the first and the second amount of said compound;
d) comparing the binding levels determined in step c), and
e) identifying said compound as rhinovirus infection treating or preventing compound if the binding level in the presence of the first amount of said compound is lower compared to the binding level in the presence of the second amount of said compound.

3. The method of claim 2, wherein a detection molecule and/or a labelling molecule is used in step c) for determining said binding levels.

4. The method of any one of claims 1 to 3, wherein said detection molecule is an anti-rhinovirus antibody or functional fragment thereof, wherein said anti-rhinovirus antibody binds to said rhinovirus strain or fragment thereof outside the rhinovirus-receptor binding domain.

5. The method of any one of claims 1 to 4, wherein said labelling molecule is covalently linked to said rhinovirus strain or fragment thereof.

6. The method of any one of claims 1 or 5, wherein said at least one type of rhinovirus receptor protein is intercellular adhesion molecule 1 (ICAM-1), low density lipoprotein receptor (LDLR), cadherin-related family member 3 (CDHR3) or a functional fragment thereof, wherein said functional fragment comprises a binding moiety for a rhinovirus strain or fragment thereof.

7. The method of any one of claims 2 to 6, wherein said rhinovirus strain or fragment thereof is pre-incubated with said compound prior to step a).

8. The method of any one of claims 2 to 7, wherein said rhinovirus receptor protein or functional fragment thereof immobilized on said solid support is pre-incubated with said compound prior to step a).

9. The method of any one of claims 2 to 8, wherein said compound is a molecule binding to said rhinovirus strain or fragment thereof.

10. The method of any one of claims 2 to 9, wherein said compound is a molecule binding to said rhinovirus receptor protein or functional fragment thereof.

11. A kit for identifying a compound for the treatment or prevention of rhinovirus infections comprising
a) a solid support coated with at least one type of rhinovirus receptor protein or a functional fragment thereof;
b) at least one rhinovirus strain or a fragment thereof; and
c) at least one detection and/or labelling molecule.

12. The kit according to claim 11, wherein said at least one type of rhinovirus receptor protein is intercellular adhesion molecule 1 (ICAM-1), low density lipoprotein receptor (LDLR), cadherin-related family member 3 (CDHR3), or a functional fragment thereof.

13. The kit according to claim 11 or 12, wherein said at least one labelling molecule is covalently linked to said rhinovirus strain or fragment thereof.

14. Use of a kit according to any one of claims 11 to 13 for identifying compounds for the treatment and prevention of rhinovirus infections.

15. Composition for the use in the treatment or prevention of viral diseases comprising at least one fragment derived from amino acid residues 28 to 110 and/or from amino acid residues 390 to 480 of ICAM-1, at least one variant of said at least one fragment or at least one antibody or functional fragment thereof binding to said at least one fragment, wherein said at least one fragment consists of 20 to 80 amino acid residues.

16. Composition for the use according to claim 15, wherein the at least one fragment derived from amino acid residues 28 to 110 of ICAM-1 comprises or consists of an amino acid sequence selected from the group consisting of SEQ ID No. 1, SEQ ID No. 2, SEQ ID No. 3 and SEQ ID No. 4 and/or the at least one fragment derived from amino acid residues 390 to 480 of ICAM-1 comprises or consists of SEQ ID No. 10.

17. An antibody directed against a C-terminal peptide of ICAM-1 for use in the treatment or prevention of a rhinovirus infection.
